(19)

Europäisches
Patentamt
European
Patent Office
Office européen
des brevets

(11) **EP 1 850 793 B1**

(12) **EUROPEAN PATENT SPECIFICATION**

(45) Date of publication and mention
of the grant of the patent:
**19.12.2018 Bulletin 2018/51**

(21) Application number: **05713306.8**

(22) Date of filing: **11.02.2005**

(51) Int Cl.:
***A61F 2/16*** (2006.01)

(86) International application number:
**PCT/US2005/004282**

(87) International publication number:
**WO 2006/088440 (24.08.2006 Gazette 2006/34)**

(54) **ASPHERIC LENSES AND LENS FAMILY**

ASPHÄRISCHE LINSEN UND LINSENFAMILIE

LENTILLES ASPHERIQUES ET GAMME DE LENTILLES

(84) Designated Contracting States:
**AT BE BG CH CY CZ DE DK EE ES FI FR GB GR
HU IE IS IT LI LT LU MC NL PL PT RO SE SI SK TR**

(43) Date of publication of application:
**07.11.2007 Bulletin 2007/45**

(73) Proprietor: **BAUSCH & LOMB INCORPORATED
Rochester, NY 14604-2701 (US)**

(72) Inventor: **ALTMANN, Griffith, E.
Pittsford, NY 14534 (US)**

(74) Representative: **Vossius & Partner
Patentanwälte Rechtsanwälte mbB
Siebertstrasse 3
81675 München (DE)**

(56) References cited:
**WO-A-2004/090611**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

Embodiments of the invention are directed to individual aspheric IOLs for use in a pseudophakic or phakic ocular system that provide specialized control of spherical aberration, and to a family of aspheric intraocular lenses (IOLs) having consistent labeling and selection parameters.

2. Description of Related Art

A simple optical system consists of a lens, which can form an image of an object. In the most basic, ideal situation, a perfect plane wavefront coming from an object located an infinite distance from the lens will be imaged to a focal point one focal length away from the lens along an optical axis of the optical system. Lens defects induce aberrations to the wavefronts of light from an object as they pass through the lens resulting in an image that is blurry.

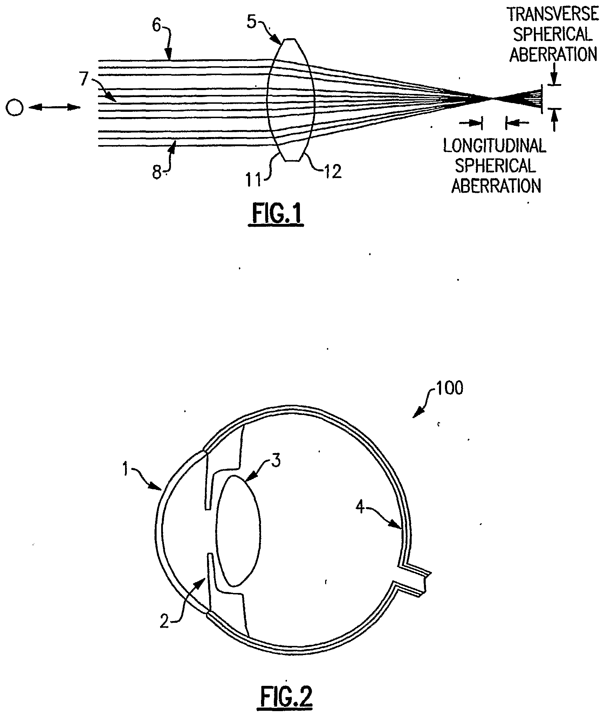

Different types of lens defects or optical system defects produce different types and degrees of aberrations that may generally appear similar to the naked eye. For example, if a perfect lens is moved along the optical axis of the optical system, the image of the object formed by the lens will suffer from defocus. Stated differently, if the surface upon which the image is viewed is moved along the optical axis, the image will likewise be defocused. The aberration of astigmatism results from in an optical system having a different focusing power in the horizontal direction than in the vertical direction, for example, resulting in a distorted image at every image location. Another troublesome aberration known as spherical aberration, illustrated in Figure 1, is produced by a lens 5 having spherical surfaces 11, 12. Light ray bundle 7 passing through the lens near its center is brought to a focus at a different position on the optical axis than the light ray bundles 6, 8 passing through the lens nearer its circumference. By convention, the spherical aberration of a lens is measured by the longitudinal or transverse distance between the center and edge focused rays of light incident on the lens as a plane wavefront originating at an optically infinite object distance, O. This is referred to as inherent spherical aberration. If a spherical lens, which by definition has inherent spherical aberration, is decentered with respect to the optical axis passing through the center of the lens, then the resulting image will be affected by other aberrations including coma and astigmatism. As mentioned above, any one or combination of these aberrations will cause the image to appear blurry, washed out or otherwise lacking in subjective quality.

The optical system of the eye is known as an ocular system, illustrated in Figure 2. In simple anatomical terms, the ocular system 100 is comprised of the cornea 1, the iris 2, the crystalline lens 3, and the retina 4. The cornea is the first component of the ocular system to receive light coming from an object and provides roughly two-thirds of the principal focusing capability of the ocular system. The crystalline lens provides the remaining focusing capability of the eye. If a plane wavefront coming from an object located at optical infinity is focused by the cornea and crystalline lens to a point in front of the retina, the eye is referred to as myopic. On the other hand, if the combined focusing power of the cornea and crystalline lens is too weak such that a plane wavefront is focused behind the retina, the ocular system is referred to as hyperopic. The function of the iris is to limit the amount of light passing through the ocular system. The crystalline lens is uniquely adapted to fine tune the focusing ability of the ocular system allowing the healthy eye to form sharp images of objects both far away and up close. The retina is the image detector of the ocular system and the interface between the eye and the brain.

As people age, the crystalline lens loses its capability to allow the ocular system to form images on the retina of near objects. Other complications, e.g., cataracts, may require that the defective crystalline lens be removed from the ocular system and a synthetic lens referred to as a pseudophakic intraocular lens (IOL) be put in its place.. Alternatively, a phakic IOL may be implanted without removing the natural crystalline lens to correct refractive errors as would be correctable with spectacles; contact lenses or corneal refractive procedures (e.g. LASIK, CK, PRK, LASEK, etc.)

Although IOLs have been around for forty plus years, they still do not provide the ocular system with the visual performance obtained with a healthy crystalline lens. This is partly due to material considerations, optical characteristics, placement accuracy and stability and other factors relating to the IOL that detract from optimal visual performance. In addition, the natural crystalline lens has aberrations with the opposite sign of those aberrations in the cornea, such that the total aberrations are reduced. This has been referred to as "aberration emmetropization". In recognition of these factors, various solutions have been developed. For example, silicone has become a favored IOL material, in addition to PMMA, hydrogels, and hydrophilic and hydrophobic acrylic materials. Scores of haptic designs have been and continue to be developed to address the positioning and stability concerns of implanted IOLs. Different surface shapes of IOLs have been provided to minimize lens weight and thickness and to control aberrations that degrade image quality. For illustration, Table 1 lists the optical prescription and technical specifications of two exemplary IOLs referred to as: LI61U, a conventional IOL with spherical anterior and posterior surfaces, manufactured by Bausch & Lomb Incorporated, Rochester, NY, and Tecnis Z9000, an advanced IOL with a prolate anterior surface and a spherical posterior surface (Advanced Medical Optics, Santa Ana, CA). In brief, the LI61U lens has positive inherent spherical aberration as with any IOL having spherical surfaces. The Tecnis Z9000 IOL has negative spherical aberration in an amount designed to offset or counter balance the positive spherical aberration of the average cornea. While both of these lenses offer certain advantages, the Tecnis Z9000 lens is directed at controlling some component of spherical aberration in the ocular system to achieve improved image quality. The intended result thus appears as one of minimizing residual spherical aberration in the image for the average population. It is well known, however, that IOLs are highly subject to movement and resulting misalignment or decentering after implantation and, that, when a lens with spherical aberration is decentered, asymmetrical aberrations such as coma and astigmatism are introduced into the image. While the effects of spherical aberration can be effectively but not completely mitigated by spectacles, the effects of coma cannot.

In view of the foregoing, the inventor has recognized the need for IOLs of alternative design that can selectively control spherical aberration, and which provide improved visual performance in ocular systems to a degree not provided by currently available lenses when used in these systems.

The availability of IOLs having different values of spherical aberration raises additional issues not heretofore dealt with in the art. Persons skilled in the art understand that an IOL is described and generally labeled for selection by two parameters: lens power and a lens constant such as, e.g., the A-constant (other lens constants may be referred to, for example, as a surgeon factor or ACD constant). Labeled lens power is expressed as the paraxial power of the lens. The paraxial power of the lens is the power of the lens through the center region of the lens very close to the optical axis. A lens having inherent spherical aberration, however, has a true power that is different than the paraxial power of the lens. For example, in a spherical lens having positive spherical aberration, the power of the lens increases as a function of radial distance away from the center of the lens. For example, using the lens prescription data for the LI61U lens from Table 3 below, the radial profile of local power and average power is as follows:

| Ray Height | Local Power (D) | Diameter | Average Power (D) |
| --- | --- | --- | --- |
| 0 | 22.00 | 0 | 22.00 |
| 0.5 | 22.05 | 1.0 | 22.02 |
| 1.0 | 22.19 | 2.0 | 22.09 |
| 1.5 | 22.43 | 3.0 | 22.21 |
| 2.0 | 22.79 | 4.0 | 22.38 |
| 2.5 | 23.27 | 5.0 | 22.61 |
| 3.0 | 23.91 | 6.0 | 22.90 |

Although this variation in power is generally, albeit imperfectly, accounted for by the various selection formulae used by surgeons for equiconvex spherical lens products, the standard formulae do not accurately account for the power variations in aspheric IOLs having inherent spherical aberration with different radial profiles.

An additional, practical concern is addressed in the following exemplary scenario. It is not uncommon for a surgeon who regularly performs IOL procedures to consistently use a limited number of IOL types or brands in their practice. For example, assume the surgeon generally prescribes the Tecnis Z9000 lens listed in Table 1 and the LI61U lens as his common alternative IOL. Each of these lens brands carries a different labeled lens (A) constant (e.g., $A_{Z9000}$ = 119; $A_{LI61U}$ = 118). Using the standard lens power equation (P = A - 2.5 L - 0.9 K, where P is the power of the IOL to be implanted, A is the A-constant of the IOL, L is the measured axial length of the eye and K is the keratometric power of the cornea; *see* below) for selecting the appropriate IOL power would indicate the use of the Tecnis Z9000 lens having a paraxial power of 23D (and inherent negative spherical aberration), or the LI61U lens having a paraxial power of 22D (and inherent positive spherical aberration). Stated differently, because these lenses will have the same shape factor to account for their spherical aberration values; i.e., they are both equiconvex), they will be labeled as having different A-constants despite both of them having a power equal to 22D. Unless the surgeon (or more typically an assistant) correctly modifies the entry of data to account for the different A constant values of the two lenses, the patient risks having an IOL implanted whose power correction is off by one diopter. Not only is the patient's resulting vision suboptimal, but there may be additional time, effort and, thus, inconvenience put on the physician.

Accordingly, as different lenses, lens families and lens brands (including those now having different spherical aberration amounts) are available for selection by the surgeon, lenses having consistently labeled parameters that inform the surgeon of the desired, correct selection, would be advantageous. The obvious advantages are the removal of guesswork on the part of the surgeon and removal of the need for the surgeon to invent new formulae to account for characteristics of the lens that may vary, such as true power and spherical aberration value. Another advantageous benefit will be realized by the lens manufacturer and pertains to various governmental approval processes for regulated products such as IOLs. For example, the approval from the US-FDA for a child-IOL having a labeled power and A-constant consistent with a parent-IOL in the exemplary case of the parent-IOL and the child-IOL having different spherical aberration values, will be considerably less burdensome and expensive than if the labeled parameters for the parent-IOL and child-IOL are necessarily different. (The term "parent-IOL" as used herein refers to an existing spherical lens or lens line identified by a labeled power and lens constant; the term "child-IOL" refers to a subsequent aspheric lens or lens line that is (or can be) labeled with the same lens power and lens constant as the parent lenses). Thus, there is a need for a family of IOLs whose individual members have characteristics that allow consistent, selection-based labeling of the lens products.

WO 2004/09611 A2 relates to an intraocular lens and method for reducing aberrations in an ocular system. At least one surface of the lens is aspherical. The lens introduces less spherical aberration than would be needed to balance undercorrection resulting from a corneal eye surface.

SUMMARY OF THE INVENTION

The invention is directed to an aspheric IOL having shape and other characteristics such that the transmission of a wavefront of light through the lens imparts no additional spherical aberration to the wavefront. As used herein, the term "shape" will specifically be referred to as "surface shape" meaning the contour or profile shape of a lens surface, or "shape factor" (defined in numerical terms below) meaning the overall shape of the lens (e.g., concave, convex, plano-convex, equiconcave, etc.). For the ocular system aspects described herein, the wavelength range of light will be the visible spectrum centered at 555nm. A non-ocular optical system can be designed to minimize aberrations over a different wavelength range. In embodiments not according to the invention, the lens has no inherent spherical aberration. In other words, a plane wavefront coming from an object at an optically infinite distance will be refracted by the lens to a sharp focal point on the optical axis of the lens. When the lens is used in an optical system having an optical axis, that includes a focusing optical element located on an object side of the lens and an image plane located on an image side of the lens, the lens will not induce any spherical aberration to a converging wavefront passing through the lens produced by the focusing element acting upon a plane wavefront incident upon the focusing element. In an aspect in which the optical system is an ocular system; i.e., the focusing element is the cornea of an eye that typically produces positive spherical aberration, the lens is an aspheric IOL that induces no additional spherical aberration to the converging wavefront incident on the IOL from the cornea. In this aspect, the IOL has a finite amount of inherent negative spherical aberration substantially less than an amount required to balance the positive spherical aberration of the cornea. According to the invention, an IOL has an inherent amount of negative spherical aberration that mimics the spherical aberration of a healthy, natural crystalline lens in a relaxed state; i.e., between about negative (-).13 micron to negative (-).07 micron of spherical aberration and, in a particular variation of this aspect, about negative (-).1 micron of spherical aberration, induced in a converging wavefront propagating from the cornea through the IOL.

A lens having no inherent spherical aberration is advantageous in that the amount of misalignment or decentering from the visual axis typically encountered in an ocular system will not induce asymmetric aberrations such as coma or astigmatism. Alternatively, although it is known that the human brain is adapted to effectively process a finite amount of positive spherical aberration in the ocular image, an aspheric IOL having a known amount of inherent negative spherical aberration is advantageous in the exemplary case of a post-LASIK myopic patient having additional positive spherical aberration induced by the LASIK procedure. According to an aspect of the embodiment, the inherent negative spherical aberration of the IOL will be limited to a range wherein the induced coma and/or astigmatism due to decentering or movement of the IOL will not exceed a predetermined value. In another aspect, an aspheric IOL having inherent positive spherical aberration will be advantageous in certain circumstances.

In an aspect, the lens has a constant ratio of a posterior apical radius of curvature to an anterior apical radius of curvature as a function of lens power. In another aspect, the ratio of an anterior surface conic constant of the lens to the posterior surface conic constant of the lens is constant for all lens radii. In a particular aspect, the ratio of anterior conic constant to posterior surface conic constant is equal to one. The apical radii will be used to influence the lens shape factor, defined as $(R_2 + R_1)/(R_2 - R_1)$, where $R_1$ and $R_2$ are the posterior and anterior apical radii, respectively.

An embodiment of the invention is directed to a family of aspheric IOLs. According to an aspect, the family of IOLs may be any two or more individual aspheric IOLs having the same labeled lens power values, different spherical aberration values, identical lens-constant values (e.g., A-constant) and different shape factors. Alternatively, the individual aspheric IOLs may have different labeled lens power values. More generally, a family may consist of lens lines A and B, each line having a different value for spherical aberration throughout the entire range of labeled lens powers for each line. In this case, the A-constant can remain the same for both the A and B line by producing each line with a different lens shape factor. Alternatively, the family of aspheric IOLs may consist of a single line of lenses having distinct discontinuous shifts in the value of spherical aberration through different ranges of labeled lens powers. In this case, the A-constant can remain the same throughout the full range of labeled powers as long as the lens shape factor is different for each range of powers with different spherical aberration values. In an embodiment not according to the invention, the family of IOLs comprises at least one IOL in a first group having an inherent negative spherical aberration value, at least one IOL in a second group having an inherent spherical aberration value substantially equal to zero and at least one IOL in a third group having an inherent positive spherical aberration value. According to an aspect, at least one of the IOLs in each of the groups has the same labeled lens power values. In the case of an ocular system in which the cornea has a typical focusing power between about 37 diopters to 49 diopters, the IOL has an inherent amount of negative spherical aberration such that no spherical aberration is induced in the converging wavefront passing through the IOL from the cornea. In a particular aspect, the amount of inherent negative spherical aberration in the IOL mimics that in a healthy crystalline lens in a relaxed state. In an alternative aspect, the IOL in the ocular system has no inherent spherical aberration, thus minimizing induced aberrations such as coma and astigmatism due to lens misalignment. In a further aspect, the IOL in the ocular system has an amount of inherent positive spherical aberration.

Also disclosed is a method for designing a family of aspheric IOLs that includes a plurality of individual aspheric IOLs each having a lens power and each having a different value of inherent spherical aberration, involving the steps of determining a lens constant that is the same for each of the plurality of individual IOLs, and providing a lens shape factor that is different for each of the plurality of individual IOLs for maintaining the same lens constant. According to an aspect, the design method provides a child-IOL or a family of child-IOLs having selection-based labeling parameters of lens power and lens constant that are the same as a respective spherical parent-IOL or family of spherical parent-IOLs, which have already received necessary approval from an appropriate governmental agency or regulating authority as the case may be.

In all of the recited embodiments, lens material may consist of silicone, PMMA, a hydrophilic acrylic, a hydrophobic acrylic, natural or artificial collagens, or urethane. Particular silicones may have an index of refraction of between 1.40 to 1.60 and, in a particular aspect, equal to about 1.43. In a particular hydrophilic acrylic aspect, the index of refraction is about 1.46.

The disadvantages, shortcomings and challenges in the current state of the art, as well as the recited objects and advantages and others are addressed and met by embodiments of the invention described below with reference to the detailed description and drawings that follow, and by embodiments of the invention as defined in the appended claims.

BRIEF DESCRIPTION OF THE DRAWINGS

- Figure 1 is a diagrammatic illustration of a spherical lens having inherent spherical aberration;
Figure 2 is a schematic illustration of a human ocular system;
Figure 3 is a schematic illustration of an aspheric IOL;
Figure 4 is a schematic illustration of an aspheric IOL;
Figures 5, 6 and 7 are MTF curves for decentering values of three comparative IOLs in a theoretical pseudophakic model eye with a 3mm pupil;
Figures 8, 9 and 10 are MTF curves for decentering values of three comparative IOLs in a theoretical pseudophakic model eye with a 4mm pupil;
Figures 11, 12 and 13 are MTF curves for decentering values of three comparative IOLs in a theoretical pseudophakic model eye with a 5mm pupil;
Figures 14, 15 and 16 are MTF curves of a Monte Carlo analysis for three comparative IOLs in a theoretical pseudophakic model eye with a 3mm, 4mm and 5mm pupil, respectively;
Figure 17 is a schematic drawing of an equiconvex spherical thick lens illustrating the principal planes of the lens;
Figure 18 is a schematic drawing of an equiconvex spherical IOL illustrating the location of the principal planes with respect to the edges of the lens;
Figure 19 is a schematic drawing of an biconvex spherical IOL illustrating the location of the principal planes as a function of lens surface radius;
Figures 20-23 are tables showing lens parameters for an equiconvex spherical lens family, a biconvex spherical lens family, a biconvex aspherical lens, and an equiconvex aspherical lens family;
Figure 24 is a graph of comparative experimental results of principal plane movement in a lens as a function of lens power for prior art spherical lenses and aspheric IOLs;
Figure 25 is a graph showing spherical aberration as a function of lens power for a prior art spherical IOL; and
Figure 26 is a comparative graph illustrating the balancing of spherical aberration and radii asymmetry as a function of lens power.

DETAILED DESCRIPTION OF A PREFERRED EMBODIMENT OF THE INVENTION

Embodiments of the invention described below relate to an aspheric lens for use in an optical system, in which the lens has physical and optical characteristics that control the spherical aberration in a wavefront passing through the lens. For the reader's clarity, the lens will be described in terms of an intraocular lens (IOL) for use in a human ocular system. In particular, the ocular system will be a pseudophakic ocular system; that is, an ocular system in which the natural crystalline lens has been removed and replaced with an implanted IOL. It is to be recognized, however, that the various embodiments of the invention apply to a phakic IOL system in which the natural crystalline lens of the ocular system has not been removed. Most generally, embodiments of the invention are directed to an aspheric lens for use in an optical system, in which the lens is designed to control spherical aberration. As used herein, the term aspheric lens refers to a lens having at least one aspheric surface that may be rotationally symmetric or asymmetric.

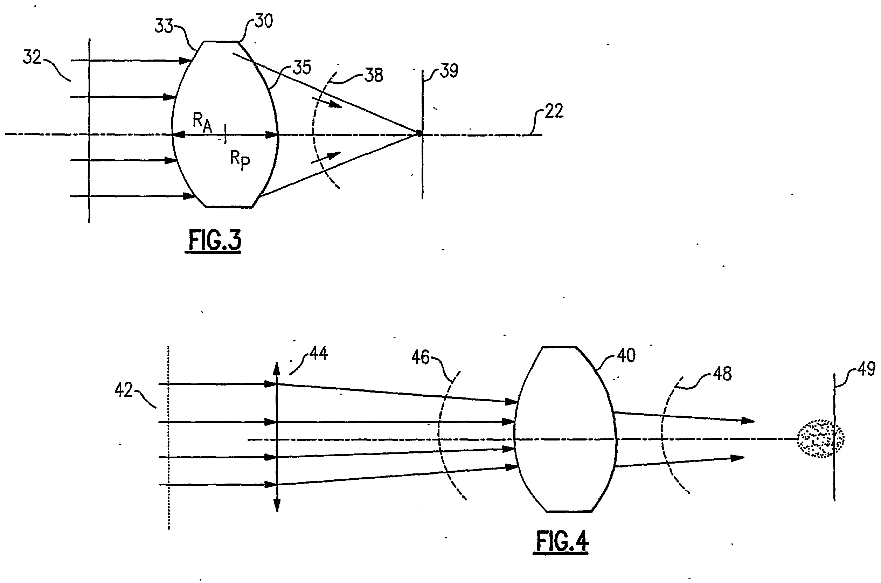

An embodiment not according to the invention is directed to an aspheric IOL characterized in that the lens has a shape factor that induces substantially no spherical aberration to a wavefront of light passing through the lens. An aspect of the embodiment is illustrated in Figure 3, which shows a plane wavefront 32 on an object side of the lens incident upon IOL 30. The IOL 30 has an anterior surface 33 and a posterior surface 35, at least one of which is an aspheric surface characterized by a conic constant and an apical radius of curvature. The lens 30 has positive optical power and focuses the wavefront 38 to a point on the optical axis at image plane 39. The lens surface asphericity is such that substantially no additional positive or negative spherical aberration is introduced into the wavefront 32 by lens 30. The lens 30 by definition has no inherent spherical aberration.

The physical characteristics of lens 30 include the apical or vertex radii of curvature, $R_a$, for the anterior surface and $R_p$ for the posterior surface, and the surface shape, or SAG, of the anterior and posterior surfaces. The SAG of an optical surface is expressed by the well-known equation

$$ SAG = (x^2/\,R_v)/1 + [1-(1+k)\,(x^2/\,R^2_v)]^{1/2} $$

where x is the radial distance from the point at which the lens surface intersects the optical axis 22 (where x equals 0) to another point on the lens surface; $R_v$ is the vertex radius of curvature of the lens surface and k is the conic constant. For a hyperbola, k < -1; for a parabola, k = -1; for a prolate ellipse, -1 < k < 0; for a sphere, k = 0; for an oblate ellipse, k > 0. Table 2 lists the physical and optical characteristics of a typical equiconvex IOL and an exemplary aspheric IOL according to an embodiment of the invention, both having a lens power of 20D. As shown in Table 2, the exemplary IOL has equal apical radii of curvature and the conic constant of both surfaces is the same. Table 3 compares the parameters of the prior mentioned spherical LI61U IOL with another exemplary spherical aberration-free aspheric IOL according to an embodiment of the invention.

In various aspects, the IOL 30 may have various shape factors including equiconvex, biconvex, plano-convex, equiconcave, biconcave or meniscus. One or both surfaces are aspheric and may or may not have the same conic constant value. Likewise, the apical radii of curvature may or may not be equal. In an exemplary aspect, the apical anterior radius, $R_A$, is not equal to the apical posterior radius of curvature, $R_p$, however the ratio of the radii remain constant over the power range of the lens.

By convention, the lens is inherently corrected for spherical aberration at a wavelength of light equal to 555nm. The lens body may be made from a biocompatible, optically transparent polymeric chemical compound such as silicone, PMMA, hydrogel, a hydrophilic or hydrophobic acrylic, natural or artificial collagen, silicone acrylic or urethane. In a particular aspect, the IOL has a lens body made of silicone having an index of refraction, n, of between 1.40 to 1.60. In a particular aspect, the lens body is made of silicone having an index of refraction of about 1.43. In another aspect, the lens body is made of a hydrophilic acrylic having an index of refraction of about 1.46. The IOL has a paraxial power of between about -10D to +40D and, more particularly, between about +15D to +40D.

The advantages of the IOL embodiment described above will now be apparent to a person skilled in the art. Since the average cornea produces approximately 0.28 micron of positive spherical aberration over the central 6 mm and a healthy natural crystalline lens in a relaxed state provides about -0.1 micron of (negative) spherical aberration, the retinal image of an object will generally have a residual amount of positive spherical aberration. The advantages of having a finite amount of residual positive spherical aberration are known to include: an increased depth of focus, which in certain circumstances may partially compensate for loss of accommodation in a presbyopic eye; positive spherical aberration may help patients with hyperopic postoperative refraction; and modest amounts of positive spherical aberration may mitigate the adverse effects of chromatic aberration and higher order monochromatic aberrations. In addition, since the IOL 30 has no inherent spherical aberration, tilting or decentering of the lens within the range of normal viewing tolerance (up to about 1mm displacement transverse to the visual axis of the eye and up to $\pm$10 degrees of rotation) will introduce a minimum amount, and perhaps no, asymmetric aberrations such as coma and/or astigmatism, which typically are induced by the misalignment of a lens with a significant amount of either positive or negative spherical aberration. Spherical aberration can be compensated with spectacle correction, but asymmetrical aberrations, like coma, cannot. Thus, in a pseudophakic ocular system including IOL 30, the resulting retinal image will have residual positive spherical aberration but no induced coma or astigmatism. An exemplary prescription of the inherent aberration free lens

is as follows:

$R_a$ = 8.014mm
$R_p$ = -10.418mm
$k_a = k_p$ = -1.085657
Center thickness (CT) = 1.29mm
Inherent spherical aberration (Z400) = 0 micron over a 5mm aperture.

When this lens is placed 4.71mm behind a perfect optical element with a power of 43D (e.g., a cornea with average power and no spherical aberration), the resulting wavefront has $0.0167\mu$ of spherical aberration. When this lens decenters 0.5mm, $0\mu$ of coma and astigmatism are induced. The exemplary lens has an effective focal length (EFL) equal to 50mm (i.e., 20D lens), an edge thickness of 0.3mm for a radial position of 3mm, and a refractive index of 1.427. The ratio between the apical radii of the anterior and posterior surfaces is -1.3 (i.e., $R_p$ = -1.3 $R_a$). The ratio between the conic constants of the anterior and posterior surfaces is 1 (i.e., $k_a = k_p$).

A study was performed using a sophisticated ray tracing program (ZEMAX, Focus Software) to evaluate the effects of lens decentration on the optical designs of three silicone IOLs in an experimental model eye: the LI61U (conventional spherical IOL), the Tecnis Z9000 (aspheric IOL) and the inherent aberration free IOL described as IOL 30 above. The study was carried out using pupil diameters of 3mm, 4mm and 5mm and lens decentrations of 0, 0.25, 0.5, 0.75 and 1.0mm. The modulation transfer functions (MTFs) were computed and plotted. A Monte Carlo simulation analysis with one thousand trials was performed with lens decentration randomly varying for each pupil size. Various reasons for lens decentration include: in-out of the bag placement, incongruency between bag diameter and overall diameter of lens, large capsulorhexis, asymmetrical capsular coverage, lens placement in sulcus, capsular fibrosis, capsular phimosis and radial bag tears. Even if the lens is perfectly centered, the other optical components of the human eye are very rarely, if ever, centered on the visual axis or any common axis. The optical performance of each IOL was evaluated in a theoretical model of a pseudophakic eye. Details about the theoretical model eye can be found in U.S. Patent Number 6,609,793. In addition, a Gaussian apodization filter was placed in the entrance pupil to simulate the Stiles-Crawford effect. In the eye model, the positive spherical aberration of the single surface model cornea matched the average value measured in recent clinical studies. The Z(4,0) Zernike coefficient for spherical aberration for the average cornea is approximately 0.28 microns over a 6mm central zone. The model eye uses an anterior chamber depth of 4.5mm, which matches the measurements of IOL axial positioning in pseudophakic eyes. The optical prescription of the model eye is given in Table 4.
In this study, each of the IOLs was a silicone lens having a power of 22D. Each lens was evaluated by centering the lens in the theoretical model eye such that the anterior surface of the IOL was 0.9mm behind the iris. For each combination of lens model and pupil diameter, the distance between the posterior surface of the IOL and the retina was optimized to obtain the best optical performance for an on-axis object located at infinity at a wavelength of 555nm. When an IOL is perfectly centered, only axial aberrations (e.g., spherical aberration) of the model cornea and the lens itself degrade the image on the model retina. Each IOL was successfully decentered in the tangential plane by 0.25mm, 0.50mm, 0.75mm and 1.0mm. The cornea, pupil and retina were always centered on the optical axis of the theoretical model eye. An array of 512 x 512 (262,144) rays was traced and the MTF was computed for each simulation. The resultant tangential and sagittal MTF curves over a spatial frequency range of 0 to 60 cycles/degree (cpd) were plotted for each simulation.

*3mm Pupil*

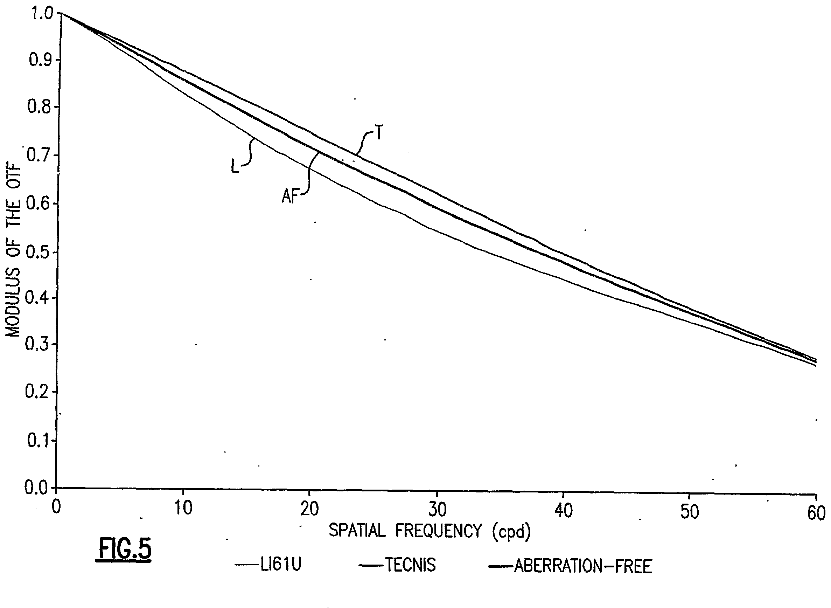

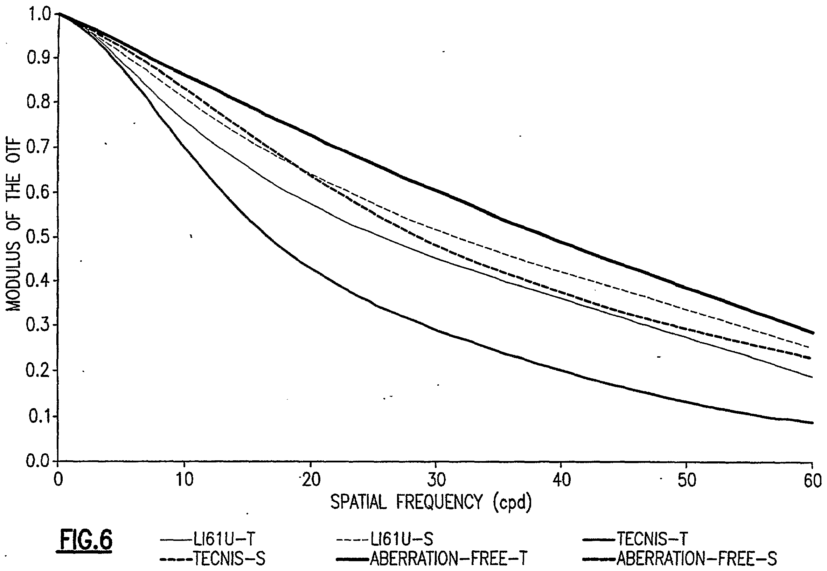

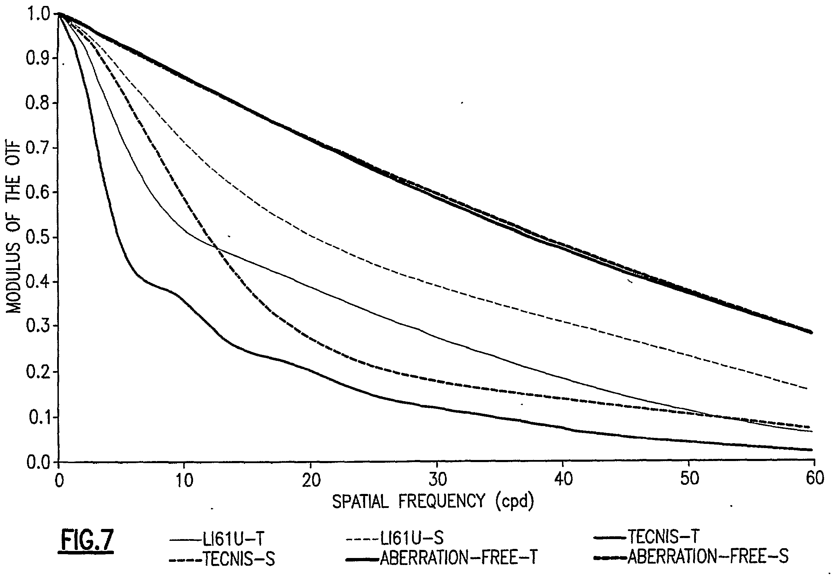

For a 3 mm. pupil, the adverse effects of the spherical aberration of the cornea and the lens are small. The Z(4,0) coefficient for corneal spherical aberration was 0.016 microns. The centered performance of the model eye with any of the three lenses is near diffraction limited, as shown in Figure 5. As the lenses decenter, the performances of the model eyes with LI61U and Z9000 lenses degrade, but the performance with the aberration free lens does not, as shown in Figures 6 and 7. Since the LI61U and Z9000 have inherent spherical aberration, higher order, asymmetrical aberrations are created when the lens is decentered, causing the tangential and sagittal MTF curves to separate and droop.
The aberration free lens was determined to outperform the LI61U over all spatial frequencies for all lens decentrations. When the aberration free lens was decentered 1mm, it continued to outperform a perfectly centered LI61U lens, and it outperformed the Z9000 lens, decentered by only 0.15 mm.

*4mm Pupil*

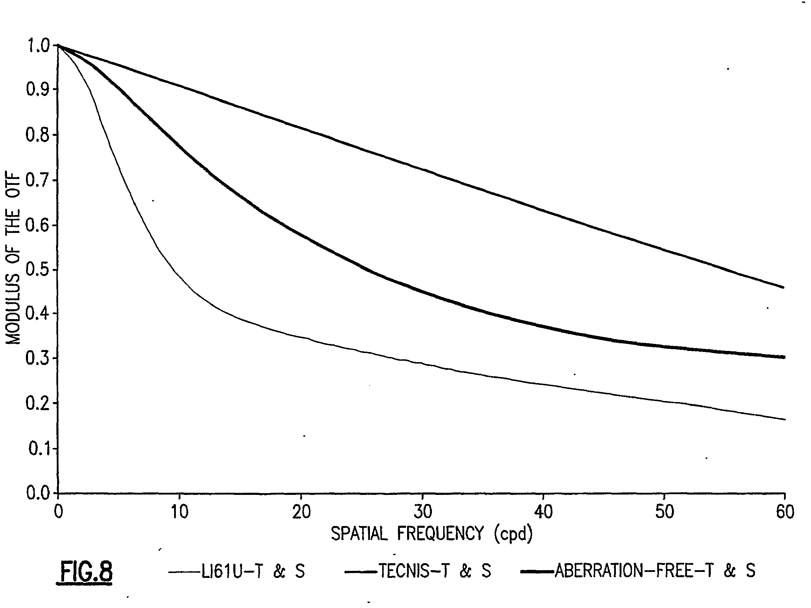

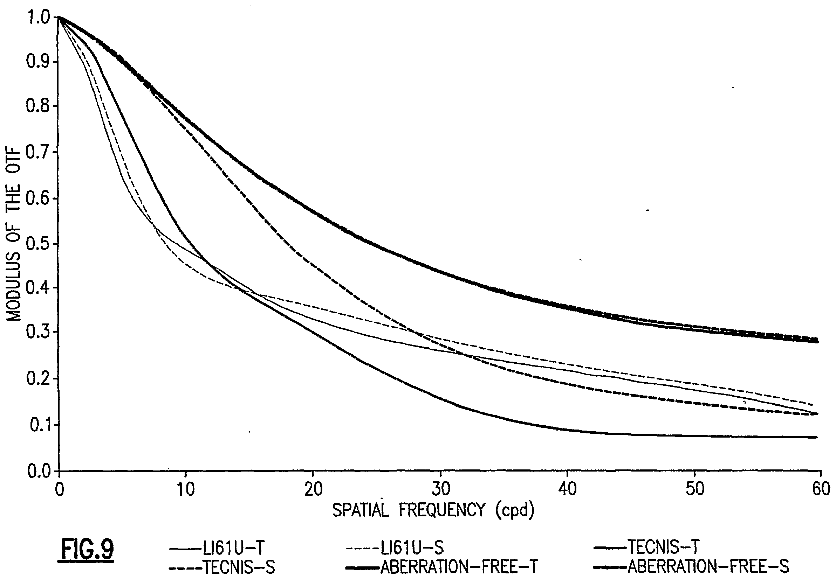

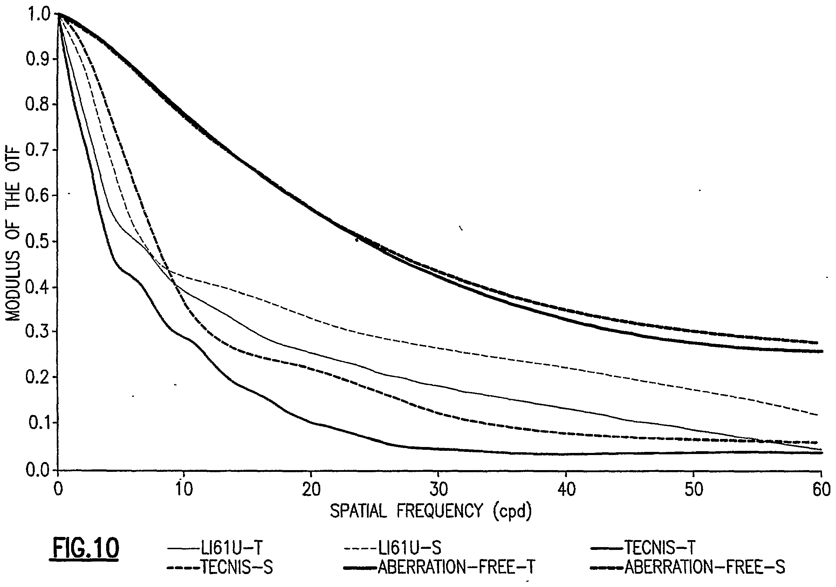

For a 4mm pupil, the adverse effects of the spherical aberration of the cornea and the lens are more problematic. The Z(4,0) coefficient for corneal spherical aberration is 0.051 micron. When the lenses are perfectly centered, the performance of the model eye with the Z9000 is diffraction limited by design as show in Figure 8. The performance of the model eye with the aberration free lens is reduced by spherical aberration of the cornea, and the performance of the LI61U is further reduced by the inherent positive spherical aberration of the lens. As the lenses are decentered, the performance of the model eyes with LI61U and Z9000 lens degrade, but the performance with the aberration free lens remains steady, as shown in Figures 9 and 10.

Similar to the 3mm pupil case, the aberration free lens outperforms the LI61U over all spatial frequencies for all lens decentrations. The aberration free lens outperforms the Z9000 lens for all spatial frequencies if the lens decentration exceeds 0.3mm. Even if the aberration free lens decentered 1mm, it outperforms the Z9000 lens decentered by only 0.3mm.

*5mm Pupil*

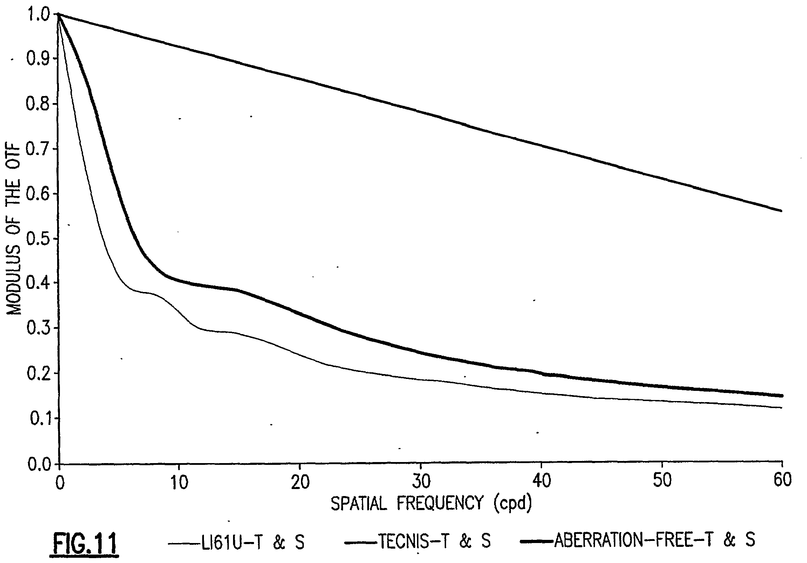

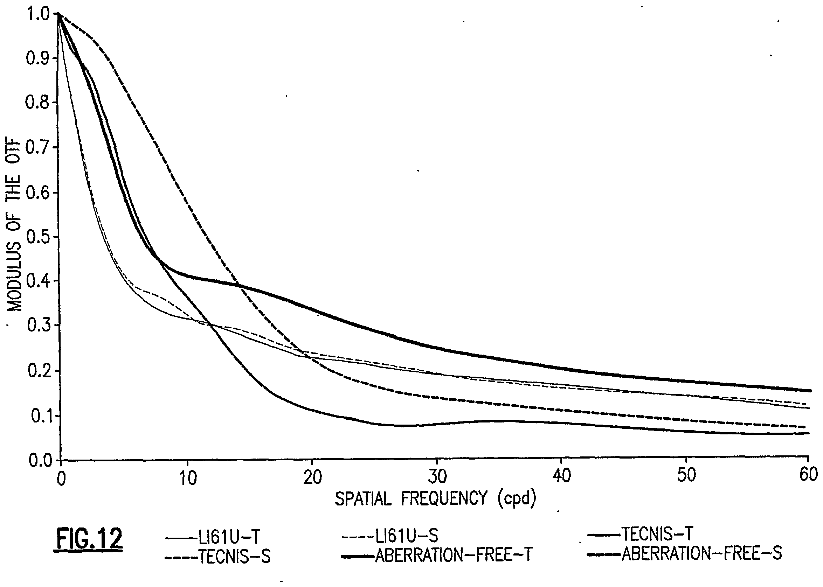

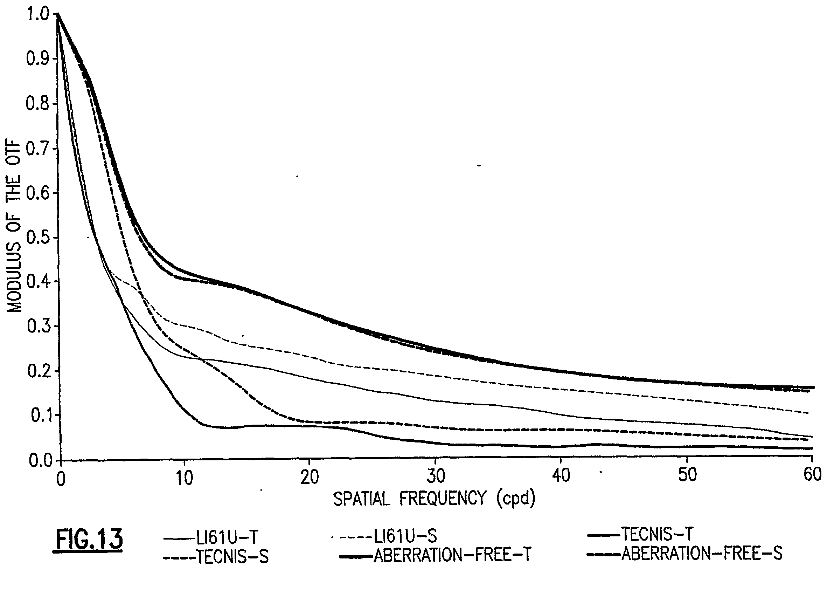

For a 5mm pupil, the adverse effects of spherical aberration of the cornea and lens are most significant. The $Z(4,0)$ coefficient for corneal spherical aberration is 0.130 micron. When the lens are perfectly centered, the performance of the model eye with the Z9000 lens is diffraction limited by design, as shown in Figure 11. The performance of the model eye with the aberration free lens is reduced by the spherical aberration of the cornea, and the performance with the LI61U is further reduced by the inherent spherical aberration of the lens. As the lenses decenter, the performance of the model eye with LI61U and Z9000 lens degrade, but the performance with the aberration free lens does not, as shown in Figures 12 and 13.

In this case, the aberration free lens outperforms the Z9000 lens if the lens decentration exceeds 0.38mm. Even if the aberration free lens is decentered 1mm, it outperforms the Z9000 lens decentered only 0.38mm.

*Monte Carlo Analysis*

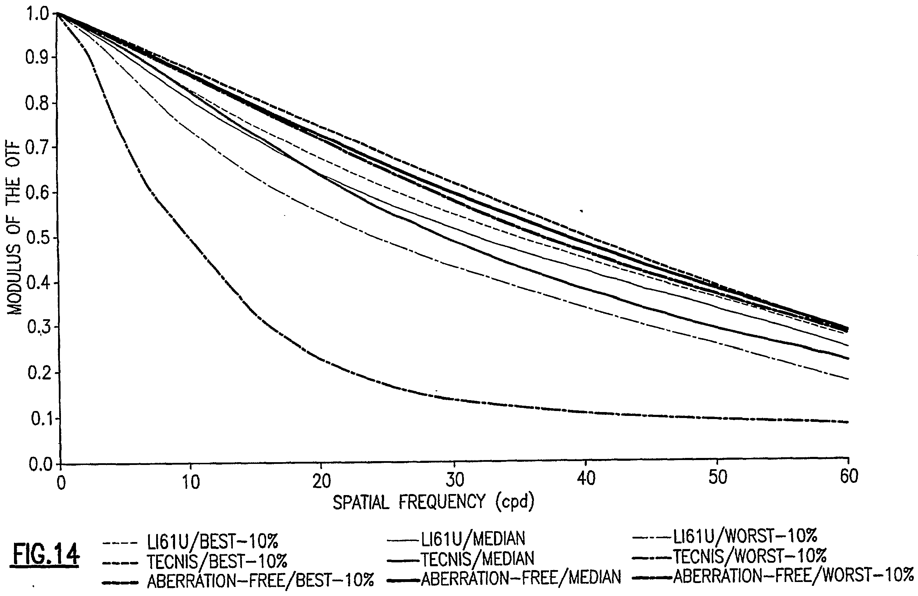

The averages of the tangential and sagittal MTF curves for 3mm, 4mm and 5mm pupil diameters are shown on Figures 14 - 16, respectively. For each lens model, the MTF curves for the worst 10 percent, best 10 percent and median cases are shown. Because the performance of the aberration free lens is independent of lens decentration, the worst 10 percent, best 10 percent and median MTF curves lie upon one another. Since the LI61U and Z9000 designs have inherent spherical aberration, their performances are dependent upon lens decentration, and thus the worst 10 percent, best 10 percent and median MTF curves are separated. Greater separation between the worst 10 percent and best 10 percent MTF curves indicates less repeatability and predictability in post-operative outcomes.

For a 3mm pupil (Figure 14), all of the MTF curves for the aberration free lens lie above the MTF curve for a perfectly centered LI61U and very nearly coincide with the best 10 percent MTF curve for the Z9000.

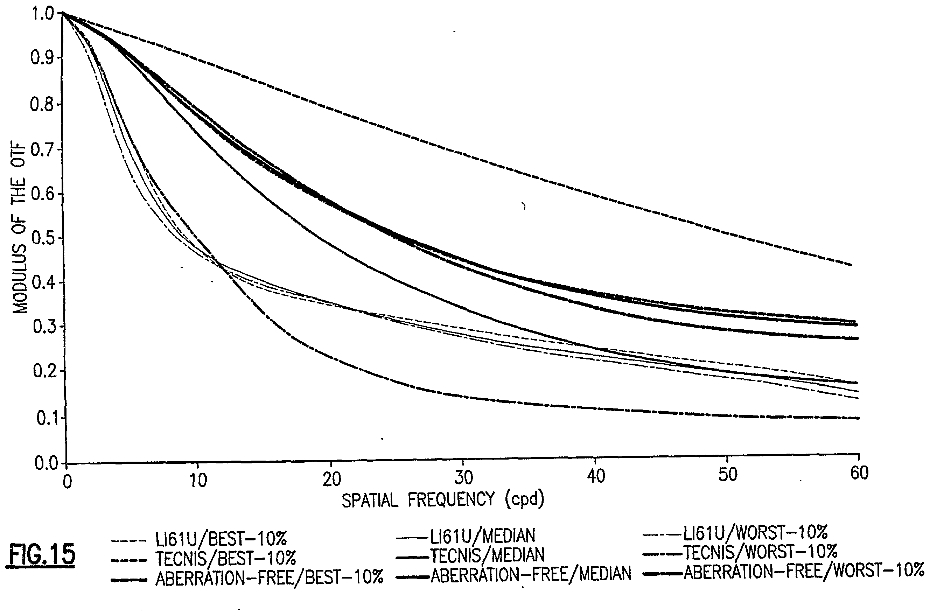

For a 4 mm pupil (Figure 15), all of the MTF curves for the aberration free lens lie above the MTF curve for a perfectly centered LI61U and the median MTF curve for the Z9000.

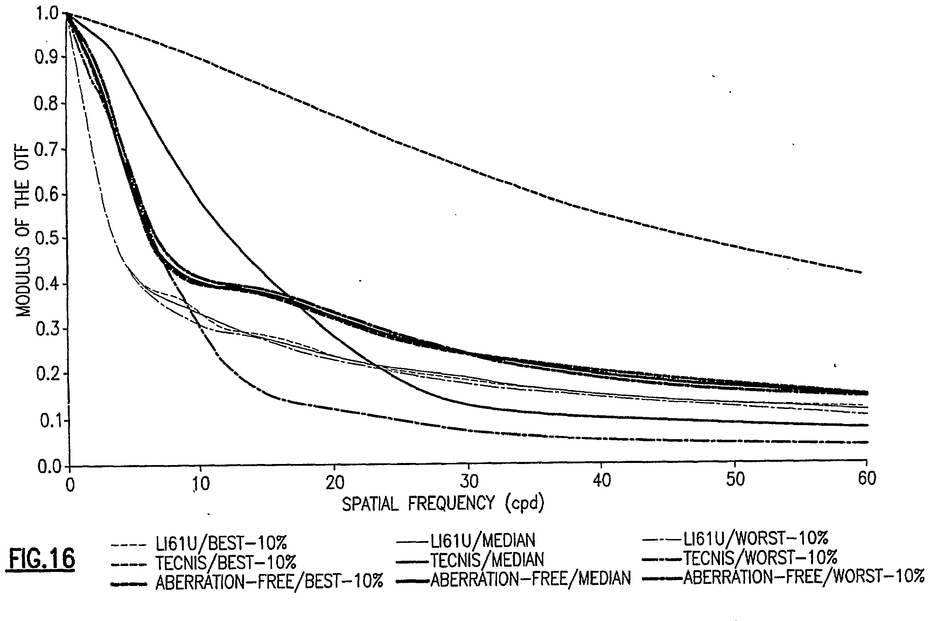

For a 5mm pupil (Figure 16), all of the MTF curves for the aberration free lens lie above the MTF curve for a perfectly centered LI61U, meaning the aberration free lens outperforms the LI61U in 100% of the cases. In the majority of cases, the aberration free lens outperforms the Z9000 for spatial frequencies greater than 17cpd.

According to another variation of the embodiment described above, an aspheric IOL has a shape that induces no spherical aberration to a converging wavefront incident from a focusing element on an object side of the lens as the wavefront passes through the IOL. Figure 4 schematically shows a pseudophakic ocular system including focusing element 44, aspheric IOL 40 and image plane 49. The focusing element 44 is representative of the cornea of the eye and image plane 49 is the retinal image plane of the ocular system. A plane wavefront 42 from an infinitely distant object is transformed into a converging wavefront 46 by the positive optical power of cornea 44. Converging wavefront 46 has positive spherical aberration induced by the cornea. The IOL 40 is characterized in that no spherical aberration is added to or subtracted from the converging wavefront 46 passing through the IOL. Thus, the converging wavefront 48 incident on the retinal image plane 49 will have a finite amount of residual positive spherical aberration produced by the cornea. In this embodiment, the IOL 40 has a small amount of negative inherent spherical aberration, such that an incident convergent wavefront will be refracted without the addition of any spherical aberration. However, the IOL 40 has substantially less negative inherent spherical aberration than the Z9000 lens referred to above. In an aspect, the aspheric IOL 40 will compensate for less than 50% of the spherical aberration created by the cornea. An exemplary prescription for the converging aberration-free lens is as follows:

$R_a$ = 8.014mm
$R_p$ = -10.418mm
$k_a = k_p$ = -1.449

Center thickness (CT) = 1.28mm (CT is reduced 10μ over aberration free lens

described above);

Inherent spherical aberration (Z400) = -0.0327 micron over a 5mm aperture.

When this lens is placed 4.71mm behind a perfect optical element with a power of 43D (e.g., a cornea with average power and no spherical aberration), the resulting wavefront has $0\mu$ of spherical aberration. However, when this lens decenters 0.5mm, only $0.016\mu$ of coma and $0.0115\mu$ of astigmatism are induced. These amounts of coma and astigmatism are small, and their adverse effects on retinal image quality will not be significant.

In a particular variation of the IOL 40, the IOL has at least one aspheric surface that induces an amount of negative spherical aberration substantially equivalent to that of a healthy natural crystalline lens in a relaxed state. Thus, the lens will induce between about - $0.13\mu$ to -$.07\mu$ of spherical aberration to a converging wavefront incident upon and refracted by the lens. In a more particular aspect, the lens surface shape is adjusted such that the lens induces about -$0.1\mu$ of spherical aberration to the converging wavefront. An exemplary prescription for the equivalent natural lens is as follows:

$R_a$ = 8.014mm
$R_p$ = -10.419mm
$k_a = k_p$= -2.698399

Center thickness (CT) = 1.2492mm (CT is reduced by 41μ over aberration free lens

described above);

Inherent spherical aberration (Z400) = -0.135 micron over a 5mm aperture.

When this lens is placed 4.71mm behind a perfect optical element with a power of 43D (e.g., a cornea with average power and no spherical aberration), the resulting wavefront has - $0.0877\mu$ of spherical aberration. However, when this lens decenters 0.5mm, $0.1428\mu$ of coma and $0.0550\mu$ of astigmatism are induced.

Another embodiment of the invention is directed to a family of aspheric IOLs. The family may consist of any two or more individual aspheric IOLs having different values of inherent spherical aberration and having a lens constant (A-constant) value that is the same for all of the lenses in the family. This can be achieved by providing a different lens shape factor for each lens having a different spherical aberration value. Different family constructs can be thought of as follows: a family may consist of a plurality of aspheric IOLs, which will have different spherical aberration values over a standard power range of -10D to 40D and more particularly over a power range of 15D to 40D. For reasons stated herein above, assume that the lens manufacturer wishes to designate this family of IOLs (the child-family) with the same A-constant as a family of standard equiconvex spherical IOLs (the parent-family) having spherical aberration values that increase as lens power increases. If the manufacturer were to keep the shape factor of the child-family of IOLs the same as the parent-family of spherical IOLs, then the A-constant should be changed, because, for each labeled paraxial power the true powers for the parent IOLs and child IOLs will be different. Hence, the manufacturer is faced with a dilemma of launching a lens with the same A-constant, which will cause post-operative refractive errors, or launch the child-family with a new A-constant (at additional labeling expense), which would cause confusion between surgeons who use both the parent spherical and child aspherical lenses. According to an embodiment of the invention, the A-constant can be maintained between the parent-family and the child-family by changing the shape factor of the child aspheric IOLs with respect to the parent spherical IOLs.

In a different scenario, a manufacturer may wish to launch a completely new family of IOLs having two or more lines (A, B, ...) where each lens line has a different value for spherical aberration. In this case, there, is no parent-family of lenses. Line A may be assumed to have a spherical aberration value of A throughout the entire range of powers, and line B having a spherical aberration value of B throughout the entire range of powers. The range of powers will be the same for both lines. If the manufacturer wishes to keep the same lens shape factor for both lines, then the A-constant will have to be different for each line, again causing potential labeling changes and surgeon confusion. However, according to an embodiment of the invention, each line of lenses may be produced with a different lens shape factor, thus maintaining the A-constant the same for both lens lines.

A further scenario may involve a new family of aspheric IOLs having only a single line of lenses, but through different ranges of powers, there are distinct discontinuous shifts in the value of spherical aberration (i.e., not the continuous increase in spherical aberration as lens power increases for spherical lenses). According to an embodiment of the invention, the A-constant can remain the same throughout the full range of powers by changing the lens shape factor for each range of powers with different spherical aberration values.

In the cases recited above, it is intended that the parent-family of IOLs or any parent lens has already obtained FDA, CE or other government regulatory agency approval such that the child-family or child lens having the same power value and A-constant will get approval more efficiently than if the labeling parameters of the child-family are different than those of the parent-family.

In an embodiment not according to the invention, a family of aspheric IOLs includes at least one aspheric IOL in a first group having an inherent negative value of spherical aberration; at least one aspheric IOL in a second group having a value of inherent spherical aberration substantially equal to zero; and at least one aspheric IOL in a third group having a value of inherent positive spherical aberration. More particularly, the value of inherent spherical aberration (i.e., the Z(4,0) Zernike coefficient using Born & Wolf notation) of the first group is in a range from less than zero to about -2.0 micron over a 6mm pupil aperture while the inherent spherical aberration in the third group is in the range of greater than zero to about 1 micron over a 6mm pupil aperture. Each group of lenses may have the same range of lens powers, but each of the at least one lenses in each group may have the same power or a different power.

According to an aspect, at least one of the aspheric IOLs in the first group having inherent negative spherical aberration is designed such that when it is used in a pseudophakic ocular system exhibiting a corneal focusing power of between about 37D to 49D, the IOL will induce no spherical aberration in a converging wavefront propagating from the cornea through the IOL. In a particular aspect, the IOL in the first group is designed so as to mimic the inherent spherical aberration of a healthy natural crystalline lens in a relaxed state such that the IOL induces between about -0.13 micron to -0.07 micron of spherical aberration to a converging wavefront of light propagating from the corneal focusing element through the lens. More particularly, the IOL will induce about -0.1 micron of spherical aberration. Thus, for all of the lenses in the first group, the resulting retinal image will have residual positive spherical aberration.

Each of the individual aspheric IOLs in the various families of lenses described herein are represented by lenses having the physical and optical characteristics of the lens embodiments described above. That is to say, each of the lenses has at least one aspheric surface characterized by a conic constant; the lens may have both anterior and posterior aspheric surfaces respectively characterized by conic constants in which the ratio of the anterior conic constant to the posterior conic constant is a constant value for all lens radii. Moreover, the apical radii of curvature of the lens play a key role in the position of the principle planes of the lens. It may be advantageous to maintain a fixed ratio between the anterior apical radius and the posterior apical radius that may or may not be equal to unity over the selected range of lens powers.

In summary, lenses described in accordance with the various embodiments of the invention control the effects of spherical aberration as a function of lens surface shape, and further, labeling characteristics of IOLs and IOL families can be made consistent between parent-families and child-families of lenses or within a family of lenses as a function of lens shape factor. The relationships between lens power, spherical aberration, lens constant and other lens variables can be further understood as follows.

As referred to above, an IOL is described by two parameters: lens power and A-constant. The extensive use of conventional equiconvex IOLs over many years enabled the development of regression formulae for selecting the power of an equiconvex IOL. The original SRK formula, developed around 1980, is

$$\text{Power} = A - 2.5\ L - 0.9\ K$$

where Power is the power of the IOL to be implanted; A is the A-constant of the IOL; L is the axial length of the eye and K is the average keratometric power of the cornea. The axial length and average keratometry values are measured prior to surgery for use in the various formulae, the most recent of which continue to use a lens constant that is directly related to the original A-constant.

Equiconvex spherical lenses have the unique property that the principal planes move very little relative to the edge of the lens throughout an exemplary power range of zero to 30D. Thus, the A-constant is nearly constant over that range of power, as will be understood by the person skilled in the art. Biconvex lenses, however, have A-constants that vary over the power range due to the different radii of curvature of the posterior and anterior surfaces. Spherical aberration, inherently present in all spherical lenses, also affects the A-constant.

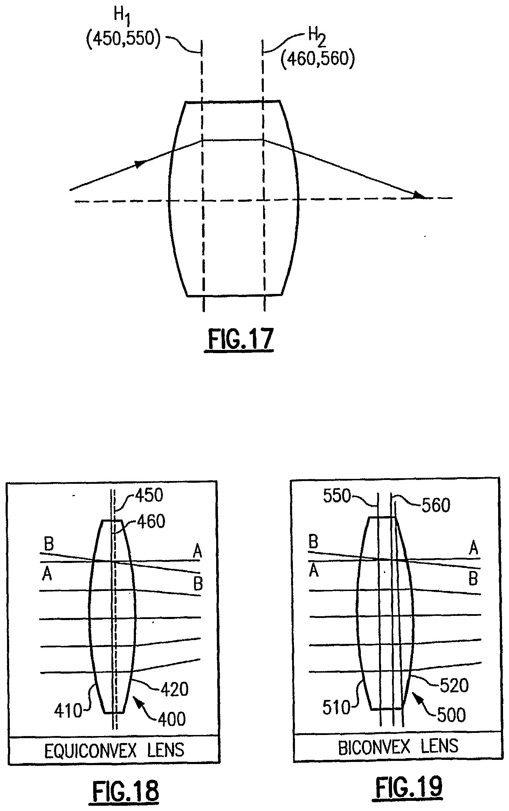

Figure 17 shows a thick lens that has first and second principal planes, HI, H2. The principal planes of a lens are hypothetical planes where all lens refraction is considered to occur. For a given lens, the principal planes are fixed and do not depend on the object position. As is known, the location of the principal planes with respect to each other and with respect to the edge location of a lens can be changed by changing the surface shape of the lens. Figures 18 and 19, respectively, show an equiconvex spherical lens 400 and a biconvex spherical lens 500. Lens 400 has first and second principal planes, 450, 460 that virtually coincide. Lens 500 has first and second principal planes 550, 560 that are separated from each other. For the equiconvex spherical lens 400, the principal planes 450, 460 are near the center of the lens because the anterior surface 410 and the posterior surface 420 have the same radius of curvature. As the radii of curvature change, the principal planes will remain substantially in the center of the lens. Thus, the A-constant of an equiconvex spherical lens remains virtually (but not entirely) constant over a wide range of powers. For the biconvex lens 500, as the radius of curvature of the posterior surface 520 increases relative to that of the anterior surface 510, the second principal plane 560 moves in the anterior direction. This will cause a change in the A-constant unless both radii of curvature are changed equally. As a result, each power of a lens and a family of biconvex spherical lenses may have a different A-constant. As referred to above, this is undesirable for the manufacturer and the physician.

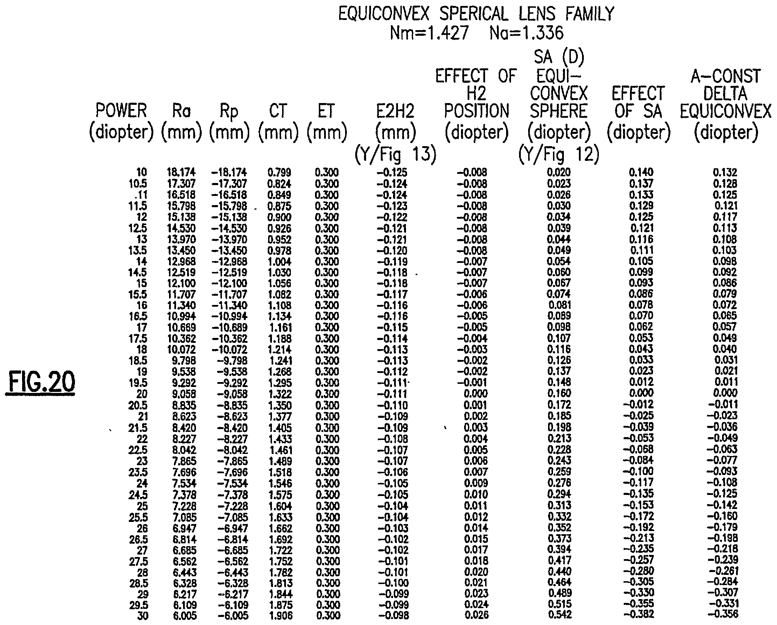

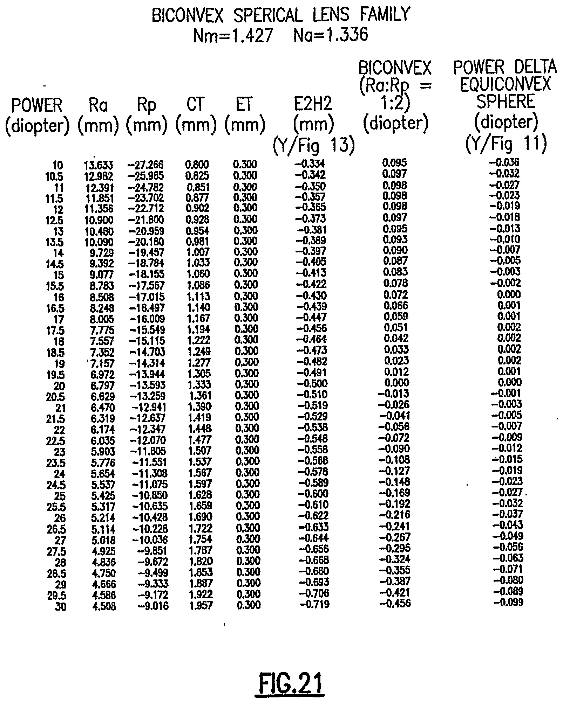

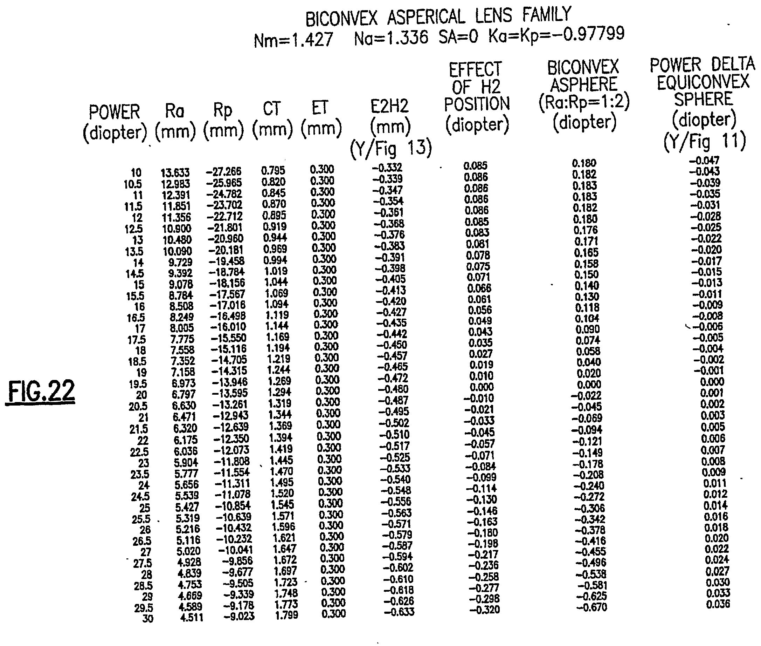

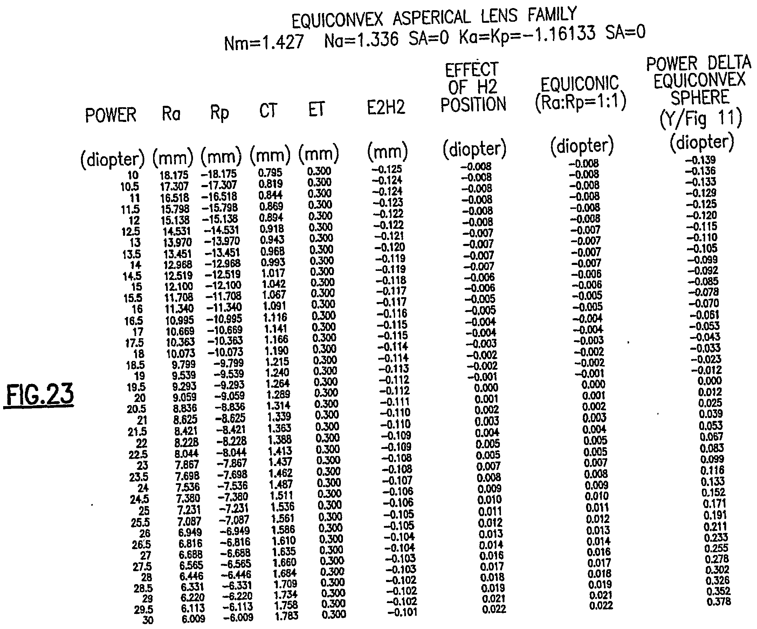

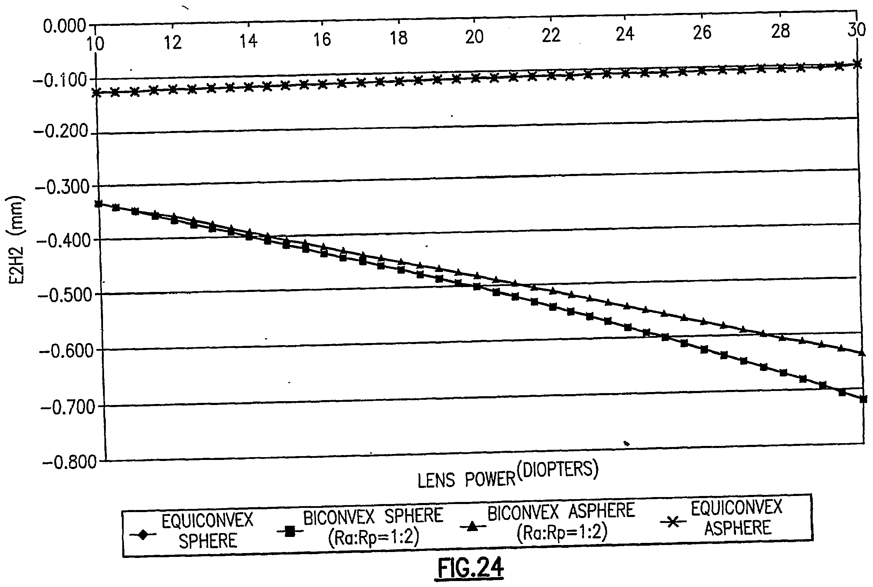

.A computer-generated experiment was made to compare the difference in the shift of the second principal plane for an equiconvex spherical lens, a biconvex spherical lens, a biconvex aspheric lens and an equiconvex aspheric lens for powers from 10D to 30D. Figure 20 shows the relevant measurement parameters for the equiconvex spherical lens; Figure 21 shows the relevant lens parameters for the biconvex spherical lens; Figure 22 shows the relevant lens parameters for the biconvex aspheric lens with anterior and posterior conic constants of (minus) -0.97799; and Figure 23 shows the relevant lens parameters for the equiconvex aspheric lens with anterior and posterior conic constants of -1.16133. Comparative experimental results are shown in Figure 24. In all of the cases, the index of refraction of the lens was 1.427 and the index of refraction of the surrounding medium (i.e., the aqueous) was 1.336. In each table of Figures 20-23, the anterior apical radius of curvature, the posterior apical radius of curvature, center thickness, edge thickness and the difference between the position of the second principal plane and the second edge (E2, H2) are listed for each paraxial power. The last column in each table shows the cumulative effect on power due to the location of the second principal plane and spherical aberration.

It can be seen from the figures that both the spherical and aspheric equiconvex lenses show little or no change in the distance between the second edge and the second principal plane. In contrast, the spherical and aspheric biconvex lenses show more dramatic changes in the location of the second principal plane with respect to the second edge. As the second principal plane H2 moves more anteriorly, the apparent power of the lens in the eye increases and vice versa. For example, if there are two lenses, A and B with the same measured power of 20D, but H2 is shifted 0.2mm anteriorly for A relative to B, then the true power of A will appear to be 0.26D stronger than B.

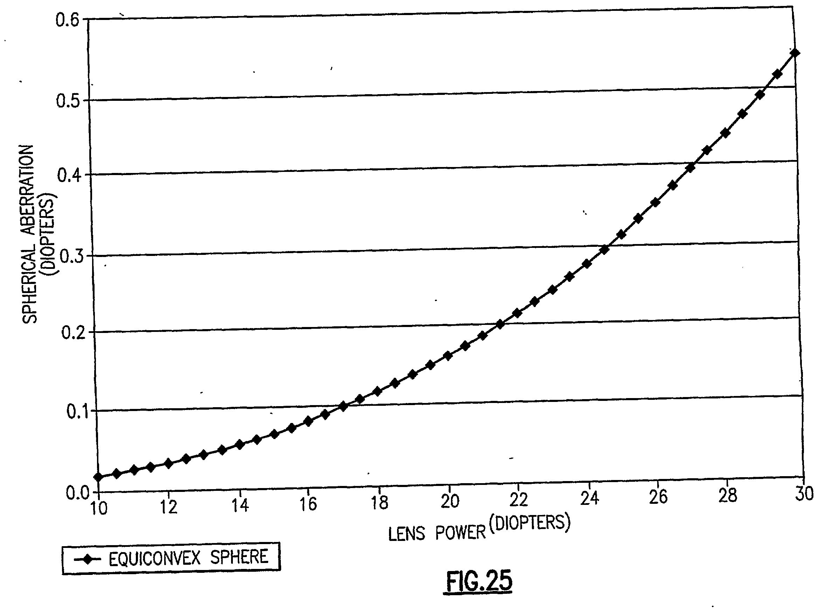

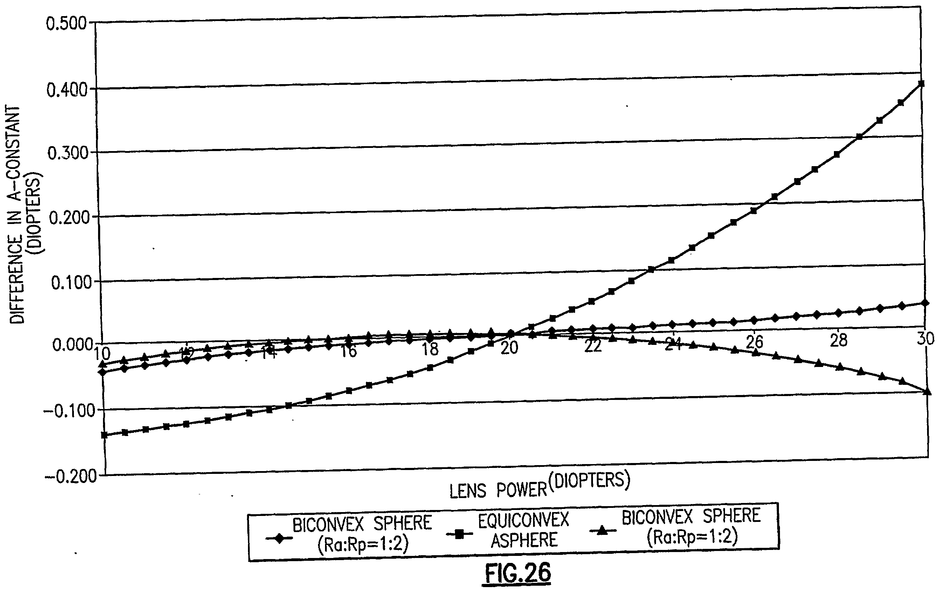

It should be noted that an aspheric lens having no inherent spherical aberration will not have the same A-constant as a spherical lens with the same lens shape factor. The effect of the spherical aberration on the A-constant is shown in Figure 25, which illustrates that the A-constant of the equiconvex spherical lens is not necessarily constant at large powers. The effects of spherical aberration and asymmetry between the anterior and posterior radii can be set to off-set or balance the changes in the A-constant, such that the *in-vivo* power of the aspheric lens will be similar to that of a parent spherical lens throughout the range of powers. In other words, an aspheric biconvex IOL can mimic the A-constant features of a spherical equiconvex IOL and provide virtually no difference between a biconvex aspheric lens and equiconvex IOL. Figure 26 illustrates the balancing of spherical aberration and radii asymmetry in order to minimize the difference in A-constant throughout the range of lens powers relative to an equiconvex design. The biconvex aspheric lens is fashioned to have even less variance in A-constant over the full range of powers. Since the A-constant of the biconvex aspheric lens can be controlled, a manufacturer may set the A-constant to be identical to the variation in the A-constant of the equiconvex lens. In effect, the A-constant of the biconvex aspheric lens can be controlled to mimic or approximate the A-constant of any known'IOL.

Disclosed is a method for designing a family of aspheric IOLs, the family including a plurality of individual aspheric IOLs each having a lens power and a different value of inherent spherical aberration, each characterized by a lens constant and a lens shape factor. The method involves the steps of determining a lens constant that is the same for each of the plurality of IOLs, and providing the lens shape factor that is different for each of the plurality of IOLs. The spherical aberration for the family may reasonably range from between about -2.0 microns to 1.0 micron over a 6mm pupil aperture. Over this range, an aspect of the design method contemplates designing lenses in groups having inherent negative spherical aberration, inherent positive spherical aberration and zero inherent spherical aberration. An aspect of the design method also includes designing at least one of the group of IOLs to induce between about -0.13 micron to -0.07 micron of spherical aberration to a converging wavefront propagating from a focusing optical element such as a cornea having a focusing power of between 37D to 49D. In another aspect, the design method contemplates designing an IOL that induces substantially no spherical aberration to a converging wavefront propagating from a focusing optical element such as a cornea.

In accordance with the family embodiments described above, each of the pluralities of IOLs is an aspheric child-lens designed such that its lens constant is the same as the lens constant of a spherical parent-lens that is not one of the family of IOLs.

The foregoing description of the preferred embodiments of the invention have been presented for the purposes of illustration and description. It is not intended to be exhaustive or to limit the invention to the precise form disclosed. Many modifications and variations are possible in light of the above teaching. It is intended that the scope of the invention be limited not by this detailed description but rather by the claims appended hereto.

**Claims**

1. An aspheric intraocular lens (IOL), comprising:

   an optical body having first and second opposing surfaces for transmitting light through the optical body; wherein both the first and second surfaces are aspheric; and wherein the IOL provides a reduced spherical aberration to a converging wavefront incident upon and refracted by the IOL, **characterised in that** the IOL has an inherent spherical aberration in the range from -0.13 to -0.07 micron over a 5 mm aperture.

2. The IOL of claim 1, wherein the ratio of a conic constant of the first surface to a conic constant of the second surface is constant for all lens radii.

3. The IOL of claim 1, wherein the ratio of a conic constant of the first surface to a conic constant of the second surface is equal to 1.

4. The IOL of claim 1, wherein the IOL has an inherent negative spherical aberration of -0.1 micron over a 5 mm aperture.

5. The IOL of claim 1, wherein the wavefront is the wavefront of light in the visible spectrum.

6. A family of aspheric intraocular lenses (IOLs), comprising two or more individual IOLs according to any of claims 1 to 5, wherein the IOLs have the same labeled lens power value, different inherent spherical aberration values, the same lens-constant value A, and different shape factors.

7. A family of aspheric intraocular lenses (IOLs), comprising two or more individual IOLs according to any of claims 1 to 5, wherein the IOLs have different labeled lens power values, different inherent spherical aberration values, the same lens-constant value A, and different shape factors.

**Patentansprüche**

1. Asphärische Intraokularlinse (IOL), mit:
   einem optischen Körper, der erste und zweite gegenüberliegende Oberflächen zum Durchlassen von Licht durch den optischen Körper aufweist; wobei sowohl die erste als auch die zweite Oberfläche asphärisch sind; und wobei die IOL eine reduzierte sphärische Aberration gegenüber einer konvergierenden Wellenfront bereitstellt, die auf die IOL einfällt und durch sie gebrochen wird, **dadurch gekennzeichnet, dass** die IOL eine inhärente sphärische Aberration im Bereich von -0,13 bis -0,07 Mikrometern über eine Apertur von 5 mm aufweist.

2. IOL nach Anspruch 1, wobei das Verhältnis einer konischen Konstante der ersten Oberfläche zu einer konischen Konstante der zweiten Oberfläche für alle Linsenradien konstant ist.

3. IOL nach Anspruch 1, wobei das Verhältnis einer konischen Konstante der ersten Oberfläche zu einer konischen Konstante der zweiten Oberfläche gleich 1 ist.

4. IOL nach Anspruch 1, wobei die IOL eine inhärente negative sphärische Aberration von -0,1 Mikrometern über eine Apertur von 5 mm aufweist.

5. IOL nach Anspruch 1, wobei die Wellenfront die Wellenfront von Licht im sichtbaren Spektrum ist.

6. Familie von asphärischen Intraokularlinsen (IOLs), die zwei oder mehrere einzelne IOLs nach einem der Ansprüche 1 bis 5 aufweisen, wobei die IOLs denselben benannten Linsenbrechungswert aufweisen, unterschiedliche inhärente sphärische Aberrationswerte, denselben Linsenkonstantenwert A und unterschiedliche Formfaktoren aufweisen.

7. Familie von asphärischen intraokularlinsen (IOLs), die zwei oder mehrere einzelne IOLs nach einem der Ansprüche 1 bis 5 aufweisen, wobei die IOLs unterschiedliche benannte Linsenbrechungswerte, unterschiedliche inhärente sphärische Aberrationswerte, denselben Linsenkonstantenwert A und unterschiedliche Formfaktoren aufweisen.

**Revendications**

1. Lentille intraoculaire (LIO) asphérique, comprenant :
   un corps optique comportant des première et seconde surfaces opposées permettant de transmettre la lumière à travers le corps optique ; dans laquelle les première et seconde surfaces sont chacune asphériques ; et dans laquelle la LIO fournit une aberration sphérique réduite à un front d'onde convergent venant frapper la LIO et réfracté par celle-ci, **caractérisée en ce que** la LIO présente une aberration sphérique inhérente allant de -0,13 à -0,07 micron sur une ouverture de 5 mm.

2. LIO selon la revendication 1, dans laquelle le rapport d'une constante conique de la première surface à une constante conique de la seconde surface est constant pour tous les rayons de lentille.

3. LIO selon la revendication 1, dans laquelle le rapport d'une constante conique de la première surface à une constante conique de la seconde surface est égal à 1.

4. LIO selon la revendication 1, dans laquelle la LIO présente une aberration sphérique négative inhérente de - 0,1 micron sur une ouverture de 5 mm.

5. LIO selon la revendication 1, dans laquelle le front d'onde est le front d'onde de la lumière dans le spectre visible.

6. Famille de lentilles intraoculaires (LIO) asphériques, comprenant au moins deux LIO individuelles selon l'une quelconque des revendications 1 à 5, dans laquelle les LIO ont la même valeur de puissance de lentille étiquetée, des valeurs d'aberration sphérique inhérente différentes, la même valeur A de constante de lentille, et des facteurs de forme différents.

7. Famille de lentilles intraoculaires (LIO) asphériques, comprenant au moins deux LIO individuelles selon l'une quelconque des revendications 1 à 5, dans laquelle les LIO ont des valeurs de puissance de lentille étiquetée différentes, des valeurs d'aberration sphérique inhérente différentes, la même valeur A de constante de lentille, et des facteurs de forme différents.

**FIG.1**

**FIG.2**

FIG.3

FIG.4

FIG.5

—LI61U     —TECNIS     —ABERRATION-FREE

EP 1 850 793 B1

FIG.6

———LI61U—T ‑‑‑‑LI61U—S ———TECNIS—T
‑‑‑‑TECNIS—S ———ABERRATION—FREE—T ———ABERRATION—FREE—S

FIG.7 ——LI61U-T ----LI61U-S ——TECNIS-T
----TECNIS-S ——ABERRATION-FREE-T ——ABERRATION-FREE-S

EP 1 850 793 B1

FIG.8 ———LI61U—T & S ——TECNIS—T & S ——ABERRATION—FREE—T & S

FIG.9

——LI61U-T ----LI61U-S ——TECNIS-T

---TECNIS-S ——ABERRATION-FREE-T --•—ABERRATION-FREE-S

**FIG.10**

— LI61U-T ----LI61U-S — TECNIS-T

----TECNIS-S — ABERRATION-FREE-T ---ABERRATION-FREE-S

EP 1 850 793 B1

FIG.11 ——LI61U–T & S ——TECNIS–T & S ——ABERRATION–FREE–T & S

EP 1 850 793 B1

FIG.12

———LI61U—T ----LI61U—S ———TECNIS—T
---TECNIS—S ■■■ABERRATION—FREE—T ▬▬▬ABERRATION—FREE—S

**FIG.13**

—— LI61U-T    · ---- LI61U-S    —— TECNIS-T
---- TECNIS-S    ━━ ABERRATION-FREE-T    ━━━ ABERRATION-FREE-S

FIG.14

---- LI61U/BEST-10%      ——LI61U/MEDIAN      ——LI61U/WORST-10%
---- TECNIS/BEST-10%     ——TECNIS/MEDIAN    ——TECNIS/WORST-10%
—— ABERRATION-FREE/BEST-10%  ——ABERRATION-FREE/MEDIAN  ——ABERRATION-FREE/WORST-10%

EP 1 850 793 B1

FIG.15

- - - - LI61U/BEST-10%        ——LI61U/MEDIAN        —·—LI61U/WORST-10%
- - - - TECNIS/BEST-10%       ——TECNIS/MEDIAN       —··—TECNIS/WORST-10%
——— ABERRATION-FREE/BEST-10%  ——ABERRATION-FREE/MEDIAN  ——ABERRATION-FREE/WORST-10%

EP 1 850 793 B1

FIG.16

- - - - LI61U/BEST-10%    ——— LI61U/MEDIAN    — - — LI61U/WORST-10%
- - - - TECNIS/BEST-10%    ——— TECNIS/MEDIAN    — - — TECNIS/WORST-10%
━━━ ABERRATION-FREE/BEST-10%  ━━━ABERRATION-FREE/MEDIAN  ━━━ABERRATION-FREE/WORST-10%

EP 1 850 793 B1

$H_1$
(450,550)

$H_2$
(460,560)

**FIG.17**

450
460

B ———————————— A
A ———————————— B

420
410 ——— 400

EQUICONVEX LENS

**FIG.18**

550 ——— 560

B ———————————— A
A ———————————— B

520
510 ——— 500

BICONVEX LENS

**FIG.19**

EQUICONVEX SPERICAL LENS FAMILY
Nm=1.427  Na=1.336

| POWER (diopter) | Ra (mm) | Rp (mm) | CT (mm) | ET (mm) | E2H2 (mm) (Y/Fig 13) | EFFECT OF H2 POSITION (diopter) | SA (D) EQUI-CONVEX SPHERE (diopter) (Y/Fig 12) | EFFECT OF SA (diopter) | A-CONST DELTA EQUICONVEX (diopter) |
|---|---|---|---|---|---|---|---|---|---|
| 10 | 18.174 | −18.174 | 0.799 | 0.300 | −0.125 | −0.008 | 0.020 | 0.140 | 0.132 |
| 10.5 | 17.307 | −17.307 | 0.824 | 0.300 | −0.124 | −0.008 | 0.023 | 0.137 | 0.128 |
| .11 | 16.518 | −16.518 | 0.849 | 0.300 | −0.124 | −0.008 | 0.026 | 0.133 | 0.125 |
| 11.5 | 15.798 | −15.798 | 0.875 | 0.300 | −0.123 | −0.008 | 0.030 | 0.129 | 0.121 |
| 12 | 15.138 | −15.138 | 0.900 | 0.300 | −0.122 | −0.008 | 0.034 | 0.125 | 0.117 |
| 12.5 | 14.530 | −14.530 | 0.926 | 0.300 | −0.121 | −0.008 | 0.039 | 0.121 | 0.113 |
| 13 | 13.970 | −13.970 | 0.952 | 0.300 | −0.121 | −0.008 | 0.044 | 0.116 | 0.108 |
| 13.5 | 13.450 | −13.450 | 0.978 | 0.300 | −0.120 | −0.008 | 0.049 | 0.111 | 0.103 |
| 14 | 12.968 | −12.968 | 1.004 | 0.300 | −0.119 | −0.007 | 0.054 | 0.105 | 0.098 |
| 14.5 | 12.519 | −12.519 | 1.030 | 0.300 | −0.118 | −0.007 | 0.060 | 0.099 | 0.092 |
| 15 | 12.100 | −12.100 | 1.056 | 0.300 | −0.118 | −0.007 | 0.067 | 0.093 | 0.086 |
| 15.5 | 11.707 | −11.707 | 1.082 | 0.300 | −0.117 | −0.006 | 0.074 | 0.086 | 0.079 |
| 16 | 11.340 | −11.340 | 1.108 | 0.300 | −0.116 | −0.006 | 0.081 | 0.078 | 0.072 |
| 16.5 | 10.994 | −10.994 | 1.134 | 0.300 | −0.116 | −0.005 | 0.089 | 0.070 | 0.065 |
| 17 | 10.669 | −10.689 | 1.161 | 0.300 | −0.115 | −0.005 | 0.098 | 0.062 | 0.057 |
| 17.5 | 10.362 | −10.362 | 1.188 | 0.300 | −0.114 | −0.004 | 0.107 | 0.053 | 0.049 |
| 18 | 10.072 | −10.072 | 1.214 | 0.300 | −0.113 | −0.003 | 0.116 | 0.043 | 0.040 |
| 18.5 | 9.798 | −9.798 | 1.241 | 0.300 | −0.113 | −0.002 | 0.126 | 0.033 | 0.031 |
| 19 | 9.538 | −9.538 | 1.268 | 0.300 | −0.112 | −0.002 | 0.137 | 0.023 | 0.021 |
| 19.5 | 9.292 | −9.292 | 1.295 | 0.300 | −0.111 | −0.001 | 0.148 | 0.012 | 0.011 |
| 20 | 9.058 | −9.058 | 1.322 | 0.300 | −0.111 | 0.000 | 0.160 | 0.000 | 0.000 |
| 20.5 | 8.835 | −8.835 | 1.350 | 0.300 | −0.110 | 0.001 | 0.172 | −0.012 | −0.011 |
| 21 | 8.623 | −8.623 | 1.377 | 0.300 | −0.109 | 0.002 | 0.185 | −0.025 | −0.023 |
| 21.5 | 8.420 | −8.420 | 1.405 | 0.300 | −0.109 | 0.003 | 0.198 | −0.039 | −0.036 |
| 22 | 8.227 | −8.227 | 1.433 | 0.300 | −0.108 | 0.004 | 0.213 | −0.053 | −0.049 |
| 22.5 | 8.042 | −8.042 | 1.461 | 0.300 | −0.107 | 0.005 | 0.228 | −0.068 | −0.063 |
| 23 | 7.865 | −7.865 | 1.489 | 0.300 | −0.107 | 0.006 | 0.243 | −0.084 | −0.077 |
| 23.5 | 7.696 | −7.696 | 1.518 | 0.300 | −0.106 | 0.007 | 0.259 | −0.100 | −0.093 |
| 24 | 7.534 | −7.534 | 1.546 | 0.300 | −0.105 | 0.009 | 0.276 | −0.117 | −0.108 |
| 24.5 | 7.378 | −7.378 | 1.575 | 0.300 | −0.105 | 0.010 | 0.294 | −0.135 | −0.125 |
| 25 | 7.228 | −7.228 | 1.604 | 0.300 | −0.104 | 0.011 | 0.313 | −0.153 | −0.142 |
| 25.5 | 7.085 | −7.085 | 1.633 | 0.300 | −0.104 | 0.012 | 0.332 | −0.172 | −0.160 |
| 26 | 6.947 | −6.947 | 1.662 | 0.300 | −0.103 | 0.014 | 0.352 | −0.192 | −0.179 |
| 26.5 | 6.814 | −6.814 | 1.692 | 0.300 | −0.102 | 0.015 | 0.373 | −0.213 | −0.198 |
| 27 | 6.685 | −6.685 | 1.722 | 0.300 | −0.102 | 0.017 | 0.394 | −0.235 | −0.218 |
| 27.5 | 6.562 | −6.562 | 1.752 | 0.300 | −0.101 | 0.018 | 0.417 | −0.257 | −0.239 |
| 28 | 6.443 | −6.443 | 1.782 | 0.300 | −0.101 | 0.020 | 0.440 | −0.280 | −0.261 |
| 28.5 | 6.328 | −6.328 | 1.813 | 0.300 | −0.100 | 0.021 | 0.464 | −0.305 | −0.284 |
| 29 | 6.217 | −6.217 | 1.844 | 0.300 | −0.099 | 0.023 | 0.489 | −0.330 | −0.307 |
| 29.5 | 6.109 | −6.109 | 1.875 | 0.300 | −0.099 | 0.024 | 0.515 | −0.355 | −0.331 |
| 30 | 6.005 | −6.005 | 1.906 | 0.300 | −0.098 | 0.026 | 0.542 | −0.382 | −0.356 |

FIG.20

## BICONVEX SPERICAL LENS FAMILY
### Nm=1.427  Na=1.336

| POWER (diopter) | Ra (mm) | Rp (mm) | CT (mm) | ET (mm) | E2H2 (mm) (Y/Fig 13) | BICONVEX (Ra:Rp = 1:2) (diopter) | POWER DELTA EQUICONVEX SPHERE (diopter) (Y/Fig 11) |
|---|---|---|---|---|---|---|---|
| 10 | 13.633 | −27.266 | 0.800 | 0.300 | −0.334 | 0.095 | −0.036 |
| 10.5 | 12.982 | −25.965 | 0.825 | 0.300 | −0.342 | 0.097 | −0.032 |
| 11 | 12.391 | −24.782 | 0.851 | 0.300 | −0.350 | 0.098 | −0.027 |
| 11.5 | 11.851 | −23.702 | 0.877 | 0.300 | −0.357 | 0.098 | −0.023 |
| 12 | 11.356 | −22.712 | 0.902 | 0.300 | −0.365 | 0.098 | −0.019 |
| 12.5 | 10.900 | −21.800 | 0.928 | 0.300 | −0.373 | 0.097 | −0.018 |
| 13 | 10.480 | −20.959 | 0.954 | 0.300 | −0.381 | 0.095 | −0.013 |
| 13.5 | 10.090 | −20.180 | 0.981 | 0.300 | −0.389 | 0.093 | −0.010 |
| 14 | 9.729 | −19.457 | 1.007 | 0.300 | −0.397 | 0.090 | −0.007 |
| 14.5 | 9.392 | −18.784 | 1.033 | 0.300 | −0.405 | 0.087 | −0.005 |
| 15 | 9.077 | −18.155 | 1.060 | 0.300 | −0.413 | 0.083 | −0.003 |
| 15.5 | 8.783 | −17.567 | 1.086 | 0.300 | −0.422 | 0.078 | −0.002 |
| 16 | 8.508 | −17.015 | 1.113 | 0.300 | −0.430 | 0.072 | 0.000 |
| 16.5 | 8.248 | −16.497 | 1.140 | 0.300 | −0.439 | 0.066 | 0.001 |
| 17 | 8.005 | −16.009 | 1.167 | 0.300 | −0.447 | 0.059 | 0.001 |
| 17.5 | 7.775 | −15.549 | 1.194 | 0.300 | −0.456 | 0.051 | 0.002 |
| 18 | 7.557 | −15.115 | 1.222 | 0.300 | −0.464 | 0.042 | 0.002 |
| 18.5 | 7.352 | −14.703 | 1.249 | 0.300 | −0.473 | 0.033 | 0.002 |
| 19 | 7.157 | −14.314 | 1.277 | 0.300 | −0.482 | 0.023 | 0.002 |
| 19.5 | 6.972 | −13.944 | 1.305 | 0.300 | −0.491 | 0.012 | 0.001 |
| 20 | 6.797 | −13.593 | 1.333 | 0.300 | −0.500 | 0.000 | 0.000 |
| 20.5 | 6.629 | −13.259 | 1.361 | 0.300 | −0.510 | −0.013 | −0.001 |
| 21 | 6.470 | −12.941 | 1.390 | 0.300 | −0.519 | −0.026 | −0.003 |
| 21.5 | 6.319 | −12.637 | 1.419 | 0.300 | −0.529 | −0.041 | −0.005 |
| 22 | 6.174 | −12.347 | 1.448 | 0.300 | −0.538 | −0.056 | −0.007 |
| 22.5 | 6.035 | −12.070 | 1.477 | 0.300 | −0.548 | −0.072 | −0.009 |
| 23 | 5.903 | −11.805 | 1.507 | 0.300 | −0.558 | −0.090 | −0.012 |
| 23.5 | 5.776 | −11.551 | 1.537 | 0.300 | −0.568 | −0.108 | −0.015 |
| 24 | 5.654 | −11.308 | 1.567 | 0.300 | −0.578 | −0.127 | −0.019 |
| 24.5 | 5.537 | −11.075 | 1.597 | 0.300 | −0.589 | −0.148 | −0.023 |
| 25 | 5.425 | −10.850 | 1.628 | 0.300 | −0.600 | −0.169 | −0.027 |
| 25.5 | 5.317 | −10.635 | 1.659 | 0.300 | −0.610 | −0.192 | −0.032 |
| 26 | 5.214 | −10.428 | 1.690 | 0.300 | −0.622 | −0.216 | −0.037 |
| 26.5 | 5.114 | −10.228 | 1.722 | 0.300 | −0.633 | −0.241 | −0.043 |
| 27 | 5.018 | −10.036 | 1.754 | 0.300 | −0.644 | −0.267 | −0.049 |
| 27.5 | 4.925 | −9.851 | 1.787 | 0.300 | −0.656 | −0.295 | −0.056 |
| 28 | 4.836 | −9.672 | 1.820 | 0.300 | −0.668 | −0.324 | −0.063 |
| 28.5 | 4.750 | −9.499 | 1.853 | 0.300 | −0.680 | −0.355 | −0.071 |
| 29 | 4.666 | −9.333 | 1.887 | 0.300 | −0.693 | −0.387 | −0.080 |
| 29.5 | 4.586 | −9.172 | 1.922 | 0.300 | −0.706 | −0.421 | −0.089 |
| 30 | 4.508 | −9.016 | 1.957 | 0.300 | −0.719 | −0.456 | −0.099 |

### FIG.21

## BICONVEX ASPERICAL LENS FAMILY

Nm=1.427   Na=1.336   SA=0   Ka=Kp=−0.97799

| POWER (diopter) | Ra (mm) | Rp (mm) | CT (mm) | ET (mm) | E2H2 (mm) (Y/Fig 13) | EFFECT OF H2 POSITION (diopter) | BICONVEX ASPHERE (Ra:Rp=1:2) (diopter) | POWER DELTA EQUICONVEX SPHERE (diopter) (Y/Fig 11) |
|---|---|---|---|---|---|---|---|---|
| 10 | 13.633 | −27.266 | 0.795 | 0.300 | −0.332 | 0.085 | 0.180 | −0.047 |
| 10.5 | 12.983 | −25.965 | 0.820 | 0.300 | −0.339 | 0.086 | 0.182 | −0.043 |
| 11 | 12.391 | −24.782 | 0.845 | 0.300 | −0.347 | 0.086 | 0.183 | −0.039 |
| 11.5 | 11.851 | −23.702 | 0.870 | 0.300 | −0.354 | 0.086 | 0.183 | −0.035 |
| 12 | 11.356 | −22.712 | 0.895 | 0.300 | −0.361 | 0.086 | 0.182 | −0.031 |
| 12.5 | 10.900 | −21.801 | 0.919 | 0.300 | −0.368 | 0.085 | 0.180 | −0.028 |
| 13 | 10.480 | −20.960 | 0.944 | 0.300 | −0.376 | 0.083 | 0.176 | −0.025 |
| 13.5 | 10.090 | −20.181 | 0.969 | 0.300 | −0.383 | 0.081 | 0.171 | −0.022 |
| 14 | 9.729 | −19.458 | 0.994 | 0.300 | −0.391 | 0.078 | 0.165 | −0.020 |
| 14.5 | 9.392 | −18.784 | 1.019 | 0.300 | −0.398 | 0.075 | 0.158 | −0.017 |
| 15 | 9.078 | −18.156 | 1.044 | 0.300 | −0.405 | 0.071 | 0.150 | −0.015 |
| 15.5 | 8.784 | −17.567 | 1.069 | 0.300 | −0.413 | 0.066 | 0.140 | −0.013 |
| 16 | 8.508 | −17.016 | 1.094 | 0.300 | −0.420 | 0.061 | 0.130 | −0.011 |
| 16.5 | 8.249 | −16.498 | 1.119 | 0.300 | −0.427 | 0.056 | 0.118 | −0.009 |
| 17 | 8.005 | −16.010 | 1.144 | 0.300 | −0.435 | 0.049 | 0.104 | −0.008 |
| 17.5 | 7.775 | −15.550 | 1.169 | 0.300 | −0.442 | 0.043 | 0.090 | −0.006 |
| 18 | 7.558 | −15.116 | 1.194 | 0.300 | −0.450 | 0.035 | 0.074 | −0.005 |
| 18.5 | 7.352 | −14.705 | 1.219 | 0.300 | −0.457 | 0.027 | 0.058 | −0.004 |
| 19 | 7.158 | −14.315 | 1.244 | 0.300 | −0.465 | 0.019 | 0.040 | −0.002 |
| 19.5 | 6.973 | −13.946 | 1.269 | 0.300 | −0.472 | 0.010 | 0.020 | −0.001 |
| 20 | 6.797 | −13.595 | 1.294 | 0.300 | −0.480 | 0.000 | 0.000 | 0.000 |
| 20.5 | 6.630 | −13.261 | 1.319 | 0.300 | −0.487 | −0.010 | −0.022 | 0.001 |
| 21 | 6.471 | −12.943 | 1.344 | 0.300 | −0.495 | −0.021 | −0.045 | 0.002 |
| 21.5 | 6.320 | −12.639 | 1.369 | 0.300 | −0.502 | −0.033 | −0.069 | 0.003 |
| 22 | 6.175 | −12.350 | 1.394 | 0.300 | −0.510 | −0.045 | −0.094 | 0.005 |
| 22.5 | 6.036 | −12.073 | 1.419 | 0.300 | −0.517 | −0.057 | −0.121 | 0.006 |
| 23 | 5.904 | −11.808 | 1.445 | 0.300 | −0.525 | −0.071 | −0.149 | 0.007 |
| 23.5 | 5.777 | −11.554 | 1.470 | 0.300 | −0.533 | −0.084 | −0.178 | 0.008 |
| 24 | 5.656 | −11.311 | 1.495 | 0.300 | −0.540 | −0.099 | −0.208 | 0.009 |
| 24.5 | 5.539 | −11.078 | 1.520 | 0.300 | −0.548 | −0.114 | −0.240 | 0.011 |
| 25 | 5.427 | −10.854 | 1.545 | 0.300 | −0.556 | −0.130 | −0.272 | 0.012 |
| 25.5 | 5.319 | −10.639 | 1.571 | 0.300 | −0.563 | −0.146 | −0.306 | 0.014 |
| 26 | 5.216 | −10.432 | 1.596 | 0.300 | −0.571 | −0.163 | −0.342 | 0.016 |
| 26.5 | 5.116 | −10.232 | 1.621 | 0.300 | −0.579 | −0.180 | −0.378 | 0.018 |
| 27 | 5.020 | −10.041 | 1.647 | 0.300 | −0.587 | −0.198 | −0.416 | 0.020 |
| 27.5 | 4.928 | −9.856 | 1.672 | 0.300 | −0.594 | −0.217 | −0.455 | 0.022 |
| 28 | 4.839 | −9.677 | 1.697 | 0.300 | −0.602 | −0.236 | −0.496 | 0.024 |
| 28.5 | 4.753 | −9.505 | 1.723 | 0.300 | −0.610 | −0.258 | −0.538 | 0.027 |
| 29 | 4.669 | −9.339 | 1.748 | 0.300 | −0.618 | −0.277 | −0.581 | 0.030 |
| 29.5 | 4.589 | −9.178 | 1.773 | 0.300 | −0.626 | −0.298 | −0.625 | 0.033 |
| 30 | 4.511 | −9.023 | 1.799 | 0.300 | −0.633 | −0.320 | −0.670 | 0.036 |

FIG.22

## EQUICONVEX ASPERICAL LENS FAMILY
### Nm=1.427   Na=1.336 SA=0   Ka=Kp=−1.16133 SA=0

**FIG.23**

| POWER (diopter) | Ra (mm) | Rp (mm) | CT (mm) | ET (mm) | E2H2 (mm) | EFFECT OF H2 POSITION (diopter) | EQUICONIC (Ra:Rp=1:1) (diopter) | POWER DELTA EQUICONVEX SPHERE (Y/Fig 11) (diopter) |
|---|---|---|---|---|---|---|---|---|
| 10 | 18.175 | −18.175 | 0.795 | 0.300 | −0.125 | −0.008 | −0.008 | −0.139 |
| 10.5 | 17.307 | −17.307 | 0.819 | 0.300 | −0.124 | −0.008 | −0.008 | −0.136 |
| 11 | 16.518 | −16.518 | 0.844 | 0.300 | −0.124 | −0.008 | −0.008 | −0.133 |
| 11.5 | 15.798 | −15.798 | 0.869 | 0.300 | −0.123 | −0.008 | −0.008 | −0.129 |
| 12 | 15.138 | −15.138 | 0.894 | 0.300 | −0.122 | −0.008 | −0.008 | −0.125 |
| 12.5 | 14.531 | −14.531 | 0.918 | 0.300 | −0.122 | −0.007 | −0.007 | −0.120 |
| 13 | 13.970 | −13.970 | 0.943 | 0.300 | −0.121 | −0.007 | −0.007 | −0.115 |
| 13.5 | 13.451 | −13.451 | 0.968 | 0.300 | −0.120 | −0.007 | −0.007 | −0.110 |
| 14 | 12.968 | −12.968 | 0.993 | 0.300 | −0.119 | −0.007 | −0.007 | −0.105 |
| 14.5 | 12.519 | −12.519 | 1.017 | 0.300 | −0.119 | −0.006 | −0.006 | −0.099 |
| 15 | 12.100 | −12.100 | 1.042 | 0.300 | −0.118 | −0.006 | −0.006 | −0.092 |
| 15.5 | 11.708 | −11.708 | 1.067 | 0.300 | −0.117 | −0.005 | −0.005 | −0.085 |
| 16 | 11.340 | −11.340 | 1.091 | 0.300 | −0.117 | −0.005 | −0.005 | −0.078 |
| 16.5 | 10.995 | −10.995 | 1.116 | 0.300 | −0.116 | −0.004 | −0.004 | −0.070 |
| 17 | 10.669 | −10.669 | 1.141 | 0.300 | −0.115 | −0.004 | −0.004 | −0.061 |
| 17.5 | 10.363 | −10.363 | 1.166 | 0.300 | −0.115 | −0.003 | −0.003 | −0.053 |
| 18 | 10.073 | −10.073 | 1.190 | 0.300 | −0.114 | −0.002 | −0.002 | −0.043 |
| 18.5 | 9.799 | −9.799 | 1.215 | 0.300 | −0.114 | −0.002 | −0.002 | −0.033 |
| 19 | 9.539 | −9.539 | 1.240 | 0.300 | −0.113 | −0.001 | −0.001 | −0.023 |
| 19.5 | 9.293 | −9.293 | 1.264 | 0.300 | −0.112 | 0.000 | 0.000 | −0.012 |
| 20 | 9.059 | −9.059 | 1.289 | 0.300 | −0.112 | 0.001 | 0.001 | 0.000 |
| 20.5 | 8.836 | −8.836 | 1.314 | 0.300 | −0.111 | 0.002 | 0.002 | 0.012 |
| 21 | 8.625 | −8.625 | 1.339 | 0.300 | −0.110 | 0.003 | 0.003 | 0.025 |
| 21.5 | 8.421 | −8.421 | 1.363 | 0.300 | −0.110 | 0.004 | 0.004 | 0.039 |
| 22 | 8.228 | −8.228 | 1.388 | 0.300 | −0.109 | 0.005 | 0.005 | 0.053 |
| 22.5 | 8.044 | −8.044 | 1.413 | 0.300 | −0.109 | 0.005 | 0.005 | 0.067 |
| 23 | 7.867 | −7.867 | 1.437 | 0.300 | −0.108 | 0.007 | 0.007 | 0.083 |
| 23.5 | 7.698 | −7.698 | 1.462 | 0.300 | −0.108 | 0.008 | 0.008 | 0.099 |
| 24 | 7.536 | −7.536 | 1.487 | 0.300 | −0.107 | 0.009 | 0.009 | 0.116 |
| 24.5 | 7.380 | −7.380 | 1.511 | 0.300 | −0.106 | 0.010 | 0.010 | 0.133 |
| 25 | 7.231 | −7.231 | 1.536 | 0.300 | −0.106 | 0.011 | 0.011 | 0.152 |
| 25.5 | 7.087 | −7.087 | 1.561 | 0.300 | −0.105 | 0.012 | 0.012 | 0.171 |
| 26 | 6.949 | −6.949 | 1.586 | 0.300 | −0.105 | 0.013 | 0.013 | 0.191 |
| 26.5 | 6.816 | −6.816 | 1.610 | 0.300 | −0.104 | 0.014 | 0.014 | 0.211 |
| 27 | 6.688 | −6.688 | 1.635 | 0.300 | −0.104 | 0.016 | 0.016 | 0.233 |
| 27.5 | 6.565 | −6.565 | 1.660 | 0.300 | −0.103 | 0.017 | 0.017 | 0.255 |
| 28 | 6.446 | −6.446 | 1.684 | 0.300 | −0.103 | 0.018 | 0.018 | 0.278 |
| 28.5 | 6.331 | −6.331 | 1.709 | 0.300 | −0.102 | 0.019 | 0.019 | 0.302 |
| 29 | 6.220 | −6.220 | 1.734 | 0.300 | −0.102 | 0.021 | 0.021 | 0.326 |
| 29.5 | 6.113 | −6.113 | 1.758 | 0.300 | −0.102 | 0.022 | 0.022 | 0.352 |
| 30 | 6.009 | −6.009 | 1.783 | 0.300 | −0.101 | | | 0.378 |

EP 1 850 793 B1

FIG.24

FIG.25

FIG.26

EP 1 850 793 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200409611 A2

- US 6609793 B